# EUROPEAN PATENT SPECIFICATION

(11) **EP 3 212 733 B1**
(45) Date of publication and mention of the grant of the patent: **28.08.2019**
(21) Application number: 15790066.3
(22) Date of filing: 27.10.2015
(51) Int. Cl.: C09K 11/06

(54) **NEW CHROMOPHORIC STRUCTURES FOR MACROCYCLIC LANTHANIDE CHELATES**
NEUE CHROMOPHORE STRUKTUREN FÜR MAKROCYCLISCHE LANTHANID-CHELATE
NOUVELLES STRUCTURES DE CHROMOPHORES POUR CHÉLATES DE LANTHANIDE MACROCYCLIQUES

(30) Priority: 29.10.2014 DK 201400627
(43) Date of publication of application: 06.09.2017
(73) Proprietor: Radiometer Turku Oy, FI-20750 Turku (FI)
(72) Inventor: TAKALO, Harri, FI-20360 Turku (FI); SUND, Henri, FI-20500 Turku (FI)
(74) Representative: Sverrisdóttir, Audur
(86) International application number: PCT/EP2015/074867
(87) International publication number: WO 2016/066641

(56) References cited:
- WO-A1-2013/011236
- WO-A1-2013/092992
- WO-A1-2014/147288
- MARINE SOULIÉ ET AL: "Comparative Analysis of Conjugated Alkynyl Chromophore-Triazacyclononane Ligands for Sensitized Emission of Europium and Terbium", CHEMISTRY - A EUROPEAN JOURNAL., vol. 20, no. 28, 17 June 2014 (2014-06-17) , pages 8636-8646, XP055240340, WEINHEIM, DE ISSN: 0947-6539, DOI: 10.1002/chem.201402415

## Description

### FIELD OF THE INVENTION

The invention relates to an azamacrocyclic lanthanide chelate design having substituted 4-(phenylethynyl)pyridine chromophores around an emitting lanthanide core. The chromophores have high molar absorptivity and luminescence with lanthanide ions. The invention also relates to the ligand from which the chelate is prepared, and to chelates attached to a biospecific reactant, and their use in various assays.

### BACKGROUND

WO2013/011236 discloses luminescent lanthanide chelates having three 4-(phenylethynyl)pyridine chromophoric groups tethered to a triazamacrocyclic core. The 4-(phenylethynyl)pyridine chromophoric groups are substituted at the para- position of the phenyl ring with an electron donating group.

The scientific literature (Tetrahedron Letters, 55, 2014, 1357-1361) acknowledges that the triazamacrocyclic ligands of the type disclosed in WO2013/011236 have relatively poor aqueous solubility. Attempts to improve aqueous solubility by appending a PEG group to the electron donating para-substituent were of limited success.

WO2013/092992 discloses luminescent lanthanide chelates having three 4-(phenylethynyl)pyridine chromophoric groups tethered to an acyclic core. In some embodiments, one chromophoric group comprises a reactive group and the other two chromophoric groups comprise two or three -OCH₂CO₂H groups in the ortho and/or para positions.

WO2014/147288 discloses triazacyclononane-based lanthanide chelate complexes useful as labelling reagents. The disclosed chelates have three 4-(phenylethynyl)pyridine chromophoric groups, one of which chromophoric groups comprises a reactive group; the other two chromophoric groups have either (i) two carboxyl (-CO₂H) substituents on the phenyl ring in the meta and para positions, or (ii) two -OCH₂CO₂H groups on the phenyl ring in the meta positions.

The article "Comparative Analysis of Conjugated Alkynyl Chromophore-Triazacyclononane Ligands for Sensitized Emission of Europium and Terbium (Chem. Eur. J. 2014, 20, 8636-8646) discloses a series of europium and terbium complexes based on a functionalized triazacyclononane carboxylate ligand. The complexes can be used in cell-imaging application but they do not possess any reactive groups to enable conjugation to a biospecific reactant.

### SUMMARY OF THE INVENTION

A first aspect of the invention relates to a luminescent lanthanide chelate of formula (I) or a salt or solvate thereof: 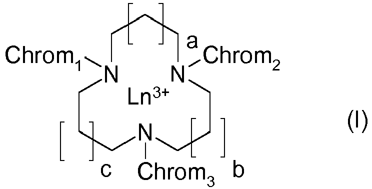 wherein a, b, and c are independently selected from 0 and 1; and
Ln³⁺ is selected from Eu^{3+,} Tb³⁺, Dy³⁺, and Sm³⁺; and
Chrom₁, Chrom₂, and Chrom₃ are of formula (II): 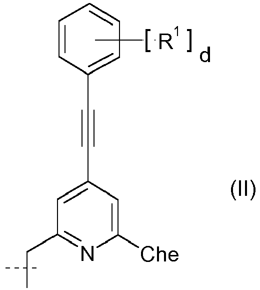
wherein Che is a chelating group independently selected from -CO₂H, -PO₃H₂,-PO(OH)R², -CH₂PO₃H₂, and -CONR³R⁴,
R² is selected from phenyl, benzyl, methyl, ethyl, propyl, n-butyl, iso-butyl, sec-butyl or tert-butyl,
R³ and R⁴ are independently selected from hydrogen and -L¹-Z¹, wherein L¹ is a direct bond or a spacer group, and Z¹ is a reactive group enabling the chelate to be linked to biospecific reactant; and
d is 1, 2, 3, 4, or 5;

R¹ is one or more substituents independently selected from any one of the group consisting of:
(i) hydrogen,
(ii) an electron donating solubilising group selected from -X-R⁵ wherein X is an oxygen atom, a sulphur atom, or-N(R⁶)CO-, R⁶ is hydrogen or -C₁₋₆alkyl, and R⁵ is selected from hydrogen, -C₁₋₆alkyl, -(CH₂)₁₋₆OH, -(CH₂)₁₋₆OC₁₋₆alkyl, -(CH₂)₁₋₆CO₂H, -(CH₂)₁₋₆CONR⁷R⁸, -(CH₂)₁₋₆SO₃H, -(CH₂)₁₋₆N(CH₃)₂, -(CH₂)₁₋₆N(CH₃)₂⁺-(CH₂)₁₋₆SO₃⁻ and polyethylene glycol, wherein R⁷ and R⁸ are each independently selected from hydrogen, C₁₋₆alkyl, -(CH₂)₁₋₆OH, -CH(CH₂OH)₂, and -CH(CH₂OH)₃,
(iii) a group selected from C₁₋₆alkyl, -(CH₂)₁₋₆OH, -(CH₂)₁₋₆OCH₃, -(CH₂)₁₋₆SCH₃
(iv) -L²-Z², wherein L² is a direct bond or a spacer group, and Z² is a reactive group enabling the chelating agent to be linked to a molecule to be labelled,
wherein two of the groups Chrom₁, Chrom₂, and Chrom₃ have two or three R¹ substituents selected from group (ii) in the para and ortho positions in relation to the acetylene group; and
wherein the third Chrom group is substituted with -L²Z².

A second aspect of the invention relates to a detectable molecule comprising a biospecific binding reagent conjugated to a luminescent lanthanide chelate according to the first aspect of the invention.

A third aspect of the invention relates to the lanthanide chelating ligand from which the chelate of the first aspect of the invention is prepared.

A fourth aspect of the invention relates to a method of carrying out a biospecific binding assay, said method comprising the steps of:
a) forming a biocomplex between an analyte and a biospecific binding reactant labelled with a luminescent lanthanide chelate according to the first aspect of the invention;
b) exciting said biocomplex with radiation having an excitation wavelength, thereby forming an excited biocomplex; and
c) detecting emission radiation emitted from said excited biocomplex.

A fifth aspect of the invention relates to a use of a detectable molecule according to the second aspect of the invention in a specific bioaffinity based binding assay utilizing time-resolved fluorometric determination of a specific luminescence-resolved fluorometric determination of a specific luminescence.

A sixth aspect of the invention relates to a solid support material conjugated with a luminescent lanthanide chelate according to the first aspect of the invention or a lanthanide chelating ligand according to the third aspect of the invention.

The lanthanide chelates and the detectable molecules of the present invention have advantageously high aqueous solubility. Detectable molecules having high aqueous solubility are useful in, for example, bioassays which benefit from a high concentration of detectable molecules. A higher concentration of detectable molecules enables a more sensitive assay, and necessitates a reduced volume of assay media. It is advantageous also because the detectable molecules have high solubility in aqueous samples requiring analysis such as blood plasma, saliva, other body fluids, and preparations thereof.

The lanthanide chelates and the detectable molecules of the present invention have advantageously high luminescence yields i.e. brightness (εΦ), especially when dry. Examples of antibodies labelled with the claimed chelate (see Examples 18 and 19) demonstrate an exceptionally high luminescence yield of up to 69500 M⁻¹cm⁻¹ when dry. This high luminescence enables a very sensitive assay because the bright biomolecule-detectable molecule conjugate is easily detected. The surprising 80-100 fold improvement in the luminescence of the dry detectable molecule compared to an aqueous solution of the same enables the skilled person to significantly increase the sensitivity of an assay by simply adding a drying step.

The ligands of the claimed invention form surprisingly stable complexes with lanthanide ions. Therefore the claimed luminescent lanthanide chelates and detectable molecules have an advantageously high stability. By 'high stability' it is meant that the complexed lanthanide ion has a reduced tendency to escape from the ligand or to be exchanged by an alternative ion. High stability is advantageous because the loss of the lanthanide ion from the ligand results in a loss of detectable luminescence, and therefore a reduced utility in the assays of the present invention. This high stability is especially useful when the chelates or detectable molecules are used in conditions having a high concentration of alternative metal ions and/or other chelates. The high stability enables the chelates of the present invention to be used together with other labelled chelates for example when two or more different probes are used in immunoassays or DNA hybridisation assays. The high stability is advantageous because the claimed chelates and detectable molecules can be used in conditions requiring an elevated temperature such as Polymerase Chain Reaction (PCR) assays, especially during the multiplication cycles. Furthermore, the chelates and detectable molecules can tolerate long incubation times in the presence of additional metal ions and/or at high temperatures.

### DETAILED DISCLOSURE OF THE INVENTION

The aim of the present invention is to provide means to obtain improved lanthanide chelate labels to be used in specific bioaffinity based binding assays, such as immunoassays (both homogeneous and heterogeneous), nucleic acid hybridization assays, receptor-binding assays, enzymatic assays, immunocytochemical, immunohistochemical assays and cell based assays utilizing fluorometric or time-resolved fluorometric determination of specific luminescence based on one or two photon-excitation. Chelates of the present invention provide means to obtain improved bioaffinity based binding assays even at wavelengths above 340 nm. The present invention makes available new ligands, chelates and detectable molecules having, for example, improved solubility, improved assay sensitivity, improved luminescence, improved high temperature stability, and improved stability in the presence of other ions and chelates.

### Luminescent lanthanide chelate

One aspect of the present invention relates to a luminescent lanthanide chelate of formula (I) or a salt thereof:

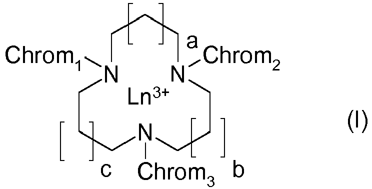

In the triazamacrocyclic ring of the present invention the units a, b, and c are independently selected from 0 and 1. In an embodiment a = b = c = 0.

Ln³⁺ is a trivalent lanthanide ion selected from europium (III) (Eu³⁺), terbium (III) (Tb³⁺), dysprosium (III) (Dy³⁺), and samarium (III) (Sm³⁺). In a preferred embodiment Ln³⁺ is Eu³⁺.

The chelates of the present invention have three chromophoric groups of formula (II), namely Chrom₁, Chrom₂, and Chrom₃.

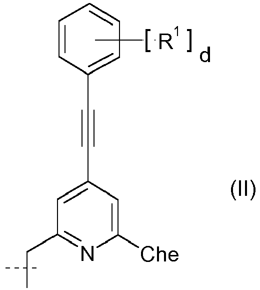

The group Che is a chelating group independently selected from -CO₂H, -PO₃H₂,-PO(OH)R², -CH₂PO₃H₂, and -CONR³R⁴ such as -CONH₂. In a preferred embodiment the group Che is -CO₂H. In embodiments where Che is ionisable, such as where Che = CO₂H, the Che group can exist in ionised (e.g. -CO₂⁻) or non-ionised (CO₂H) forms.

The group R² is selected from phenyl, benzyl, methyl, ethyl, propyl, n-butyl, iso-butyl, sec-butyl or tert-butyl.
R³ and R⁴ are independently selected from hydrogen and -L¹-Z¹, wherein L¹ is a direct bond or a spacer group, and Z¹ is a reactive group enabling the chelate to be linked to a molecule biospecific reactant. In an embodiment, the groups R³ and R⁴ are both hydrogen.

The phenyl rings of the chromophoric groups Chrom₁, Chrom₂, and Chrom₃ are each substituted with 1, 2, 3, 4, or 5 R¹ groups.

The 1, 2, 3, 4 or 5 R¹ groups are each individually selected from any one of the groups consisting of:
(i) hydrogen,
(ii) an electron donating solubilising group selected from -X-R⁵ wherein X is an oxygen atom, a sulphur atom, or-N(R⁶)CO-, R⁶ is hydrogen or C₁₋₆alkyl such as methyl, ethyl, propyl, isopropyl, n-butyl, sec-butyl or t-butyl, and R⁵ is selected from hydrogen, -C₁₋₆alkyl such as methyl, ethyl, propyl, isopropyl, n-butyl, sec-butyl or t-butyl, -(CH₂)₁₋₆OH such as -CH₂OH or -(CH₂)₂OH, -(CH₂)₁₋₆OC₁₋₆alkyl such as -(CH₂)OCH₃ or-(CH₂)₂OCH₃, -(CH₂)₁₋₆CO₂H such as -CH₂CO₂H or -(CH₂)₂CO₂H, -(CH₂)₁₋₆CONR⁷R⁸ such as -CH₂CONH₂, -(CH₂)₁₋₆SO₃H, -(CH₂)₁₋₆N(CH₃)₂, -(CH₂)₁₋₆N(CH₃)₂⁺-(CH₂)₁₋₆SO₃⁻ and polyethylene glycol such as -(CH₂CH₂O)₁₋₄OCH₂CH₂OH, or -(CH₂CH₂O)₁₋₄OCH₂CH₂OCH₃;
   wherein Rand R⁸ are each independently selected from hydrogen, C₁₋₆alkyl, such as methyl, ethyl, propyl, isopropyl, n-butyl, sec-butyl or t-butyl, -(CH₂)₁₋₆OHsuch as -CH₂OH or -(CH₂)₂OH, -CH(CH₂OH)₂, and -CH(CH₂OH)₃,
   in one embodiment an electron donating solubilising group selected from -X-R⁵ wherein X is an oxygen atom or-N(R⁶)CO-, R⁶ is hydrogen or C₁₋₆alkyl, and R⁵ is selected from hydrogen, -(CH₂)₁₋₆OH, -(CH₂)₁₋₆CO₂H, -(CH₂)₁₋₆CONR₇R₈, and -(CH₂)₁₋₆SO₃H, wherein R⁷ and R⁸ are each independently selected from hydrogen, C₁₋₆alkyl-OH, -CH(CH₂OH)₂, and -CH(CH₂OH)₃,
(iii) a group selected from C₁₋₆alkyl such as methyl, ethyl, propyl, isopropyl, n-butyl, sec-butyl or t-butyl, -(CH₂)₁₋₆OHsuch as -CH₂OH or -(CH₂)₂OH, -(CH₂)₁₋₆OCH₃ such as-(CH₂)OCH₃ or -(CH₂)₂OCH₃, or -(CH₂)₁₋₆SCH₃,
   in one embodiment a group selected from C₁₋₆alkyl such as methyl, ethyl, propyl, isopropyl, n-butyl, sec-butyl or t-butyl, -(CH₂)₁₋₆OHsuch as -CH₂OH or -(CH₂)₂OH,
(iv) -L²-Z², wherein L² is a direct bond or a spacer group, and Z² is a reactive group enabling the chelating agent to be linked to a biospecific reactant.

Two of the groups Chrom₁, Chrom₂, and Chrom₃ have two or three R¹ substituents selected from group (ii) in the para and ortho positions in relation to the acetylene group, and the third Chrom group is substituted with -L²-Z².

In an embodiment X is an oxygen atom. In a preferred embodiment X is an oxygen atom and R⁵ is -(CH₂)₁₋₆CO₂H such as -CH₂CO₂H, or -(CH₂)₁₋₆SO₃H, or -(CH₂)₁₋₆N(CH₃)₂⁺-(CH₂)₁₋₆-SO₃⁻.

In an embodiment at least one of the chromophoric groups Chrom₁, Chrom₂, and Chrom₃ are independently selected from the chromophoric groups of formula (Ila), (IIb) or (IIc) in which the groups R^{1A}, R^{1AA}, R^{1AAA}, R^{1B}, R^{1BB}, R^{1C}, and R^{1CC} are each independently selected from R¹ group (ii) as defined hereinbefore.

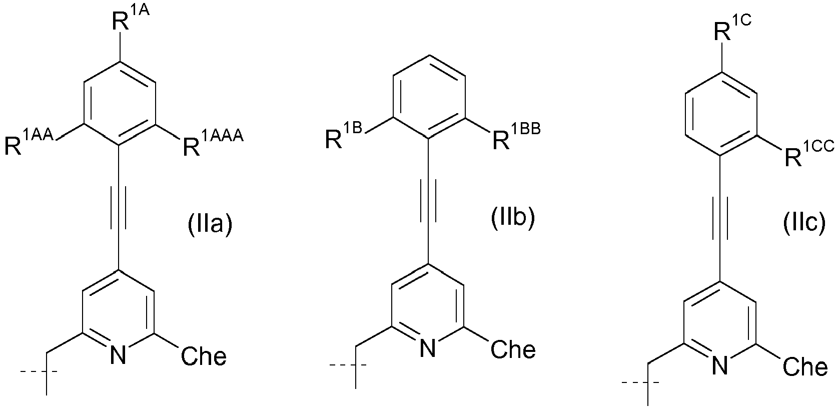

In a preferred embodiment the groups R^{1A}, R^{1AA}, R^{1AAA}, R^{1B}, R^{1BB}, R^{1C}, and R^{1CC} are - OCH₂CO₂H.

In a preferred embodiment the chelating agents of formula (I) have only one reactive group. In a preferred embodiment the Che group does not comprise a reactive group. Rather, the reactive group Z² is connected via L² to the phenyl ring of a chromophoric group selected from Chrom₁, Chrom₂, and Chrom₃.

In a preferred embodiment, two of the chromophoric groups Chrom₁, Chrom₂, and Chrom₃ are selected from formula (IIa), (IIb) or (IIc) as defined hereinbefore, and the third chromophoric group is selected from (IId), (IIe), or (IIf): 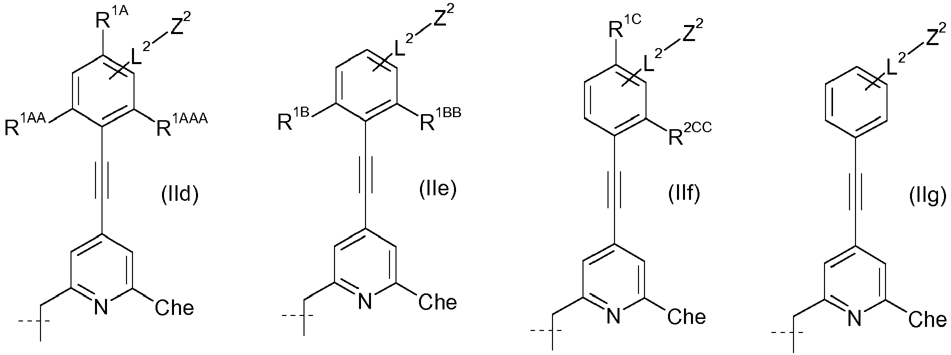 wherein R^{1A}, R^{1AA}, R^{1AAA}, R^{1B}, R^{1BB}, R^{1C}, and R^{1CC} are each independently selected from R¹ group (ii) as defined hereinbefore. In a preferred embodiment L² is a direct bond and Z² is an isothiocyanato (-NCS) group. In a preferred embodiment the chromophoric group comprising the reactive group has formula (IIg).

As used herein, the term C₁₋₆alkyl includes, but is not limited to, the following alkyl groups: methyl, ethyl, propyl, isopropyl, n-butyl, sec-butyl and t-butyl.

As used herein, the terms 'ortho' and 'para' when used to describe the substitution pattern of a 6-membered ring (e.g. phenyl) mean substituted at the 2- and 4- positions respectively. For example, a phenyl ring substituted with three substituents at the ortho and para positions is a 2, 4, 6 substituted ring.

It should be understood that when the ligands and chelates of the present invention comprise ionisable groups such as carboxylates, sulfonates and the like, the chelates may be present in ionised (e.g. -CO₂⁻) or non-ionised (e.g. -CO₂H) forms, and if ionised may include cations as counter ions, e.g. Na+, K+, Ca2+ and the like.

The chelates and ligands of the present invention comprise a reactive group (Z¹ or Z²), optionally linked to the ligand or chelate by a spacer (L¹ or L²). In such instances, the reactive group is facilitating the labelling of a biospecific binding reactant, or is facilitating the formation of a covalent bond to a solid support material. In case the chelate has a polymerizing group as reactive group, then the chelate may be introduced in the solid support, e.g. a particle, simultaneously with the preparation of the particles.

If present, the reactive group is typically selected from azido (-N₃), alkynyl (-C=CH), alkylene (-CH=CH₂), amino (-NH₂), aminooxy (-O-NH₂), hydrazide (-CONHNH₂), aldehyde (-CHO), mercapto (-SH), maleimido, activated derivatives of maleimido, isocyanato (-NCO), isothiocyanato (-NCS), diazonium (-N⁺N), bromoacetamido, iodoacetamido, reactive esters, pyridyl-2-dithio, and 6-substituted 4-chloro-1,3,5-triazin-2-ylamino, in particular, the reactive group comprises a isothiocyanato (-NCS) group.

The substituents in 6-substituted 4- chloro-1,3,5-triazin-2-ylamino can be selected from the group consisting hydrogen, halogen, alkoxy, aryloxy, amino, alkyl with one to six carbon atoms, substituted amino or thioethers, and preferable selected from the group consisting of chloro, fluoro, ethoxy, 2-methoxyethoxy, 2-cyanoethoxy, 2,2,2-trifluoroethoxy, thiophenoxy or ethoxycarbonylthiomethoxy. The substituted amino or thioether is preferable mono- or disubstituted each substituent being preferable independently selected from C₁₋₆-alkyl, C₁₋₆-alkyl-O-, phenyl, carbonyl or carboxyl.

It follows that upon reaction with a biospecific binding reactant, the reactive group establishes a link to said biospecific binding reactant, e.g. of one of the following types: a thiourea (-NH-C(=S)-NH-), an aminoacetamide (-NH-CO-CH₂-NH-), an amide (-NH-CO-, -CO-NH-, -NCH₃-CO- and -CO-NCH₃-), oxime (-O-N=CH-), hydrazone (-CO-NH-NH=CH-) (and aliphatic thioether (-S-), a disulfide (-S-S-), a 6-substituted-1,3,5-triazine-2,4-diamine, a 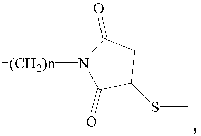 wherein n = 1-6; and a triazole (e.g. formed by the so-called "click" chemistry).

It should be understood that when a reactive group (e.g. Z¹ or Z²) is present, the group may include a spacer (e.g. L¹ or L²), i.e. a distance-making biradical, so as - if necessary or desirable - to position the reactive group in a position accessible for reaction with the biospecific binding reactant. The spacer may be readily introduced in the course of the synthesis of the ligand or the chelate.

The term "spacer" is intended to mean a distance-making group between, e.g., a conjugating group or a pyridine moiety of the core structure and, e.g. the reactive group. The spacer typically has a length of 1-20 bonds between the attachment point and reactive group, such as 3-15 bonds, or 5-12 bonds. The said spacer is formed of one to five moieties, each moiety selected from the group consisting of phenylene, alkylene containing 1-10 carbon atoms, an ethynediyl (-C=C-), an ether (-O-), a thioether (-S-), a disulfide (-S-S-), an amide (-C(=O)-NH-, -NH-C(=O)-, -C(=O)-NCH₃- and -NCH₃-C(=O)-), a thiourea (-NH-C(=S)-NH-) and a triazole.

### Particular embodiments

In an preferred embodiment, the chelate of the present invention has formula (IIIa) wherein R^{1AA} is hydrogen or -OCH₂CO₂⁻:

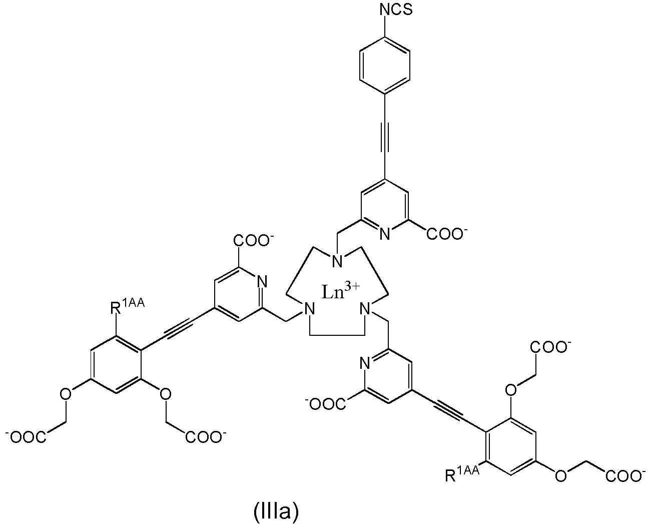

In another preferred embodiment, the chelate of the present invention has the formula (IIIb)

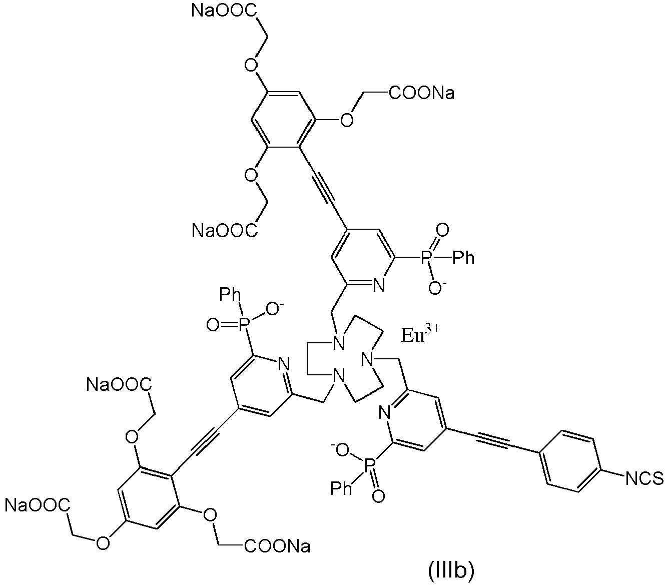

### Lanthanide chelating ligand

Another aspect of the invention relates to a lanthanide chelating ligand of formula (IV) wherein a, b, c, Chrom₁, Chrom₂, and Chrom₃ are as defined for formula (I).

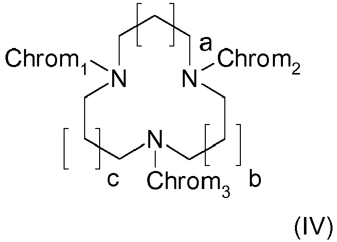

### A detectable molecule

Still another aspect of the present invention relates to a detectable molecule comprising a biospecific binding reactant conjugated to a luminescent lanthanide chelate as defined hereinabove. Conjugation is typically obtained by means of a reactive group of said chelate.

The biospecific binding reactant should be capable of specifically binding an analyte of interest for the purpose of quantitative or qualitative analysis of said analyte in a sample.

Examples of biospecific binding reactants are those selected from an antibody, an antigen, a receptor ligand, a specific binding protein, a DNA probe, a RNA probe, an oligopeptide, an oligonucleotide, a modified oligonucleotide (e.g. an LNA modified oligonucleotide), a modified polynucleotide (e.g. an LNA modified polynucleotide), a protein, an oligosaccaride, a polysaccharide, a phospholipid, a PNA, a steroid, a hapten, a drug, a receptor binding ligand, and lectine. In a preferred embodiment, the biospecific binding reactant is selected from antibodies, e.g. Troponin I antibodies (anti-Tni).

### A method for carrying out a biospecific binding assay

A still further aspect of the invention relates to a method of carrying out a biospecific binding assay, wherein the method comprises the steps of:
a) forming a biocomplex between an analyte and a biospecific binding reactant labelled with a lanthanide chelate as defined herein;
b) exciting said biocomplex with radiation having an excitation wavelength, thereby forming an excited biocomplex; and
c) detecting emission radiation emitted from said excited biocomplex.

In step b), the excitation wavelength is preferably 300 nm or longer, e.g. around 320-360
nm.
The method follows the conventional assay steps as will be evident for the skilled person.

This being said, a further aspect of the invention relates to the use of a detectable molecule as defined above in a specific bioaffinity based binding assay utilizing time-resolved fluorometric determination of a specific luminescence based on one or two photon-excitation. In one embodiment, the specific bioaffinity based binding assay is a heterogeneous immunoassay, a homogenous immunoassay, a DNA hybridization assay, a receptor binding assay, an immunocytochemical or an immunohistochemical assay.

In an alternative embodiment, one or more of steps a), b), and c) is performed at an elevated temperature such as above 40°C, above 50°C, above 60°C, above 70°C, above 80°C, above 90°C or above 100°C. In an embodiment step, step a) (i.e the formation of the biocomplex) is performed at an elevated temperature as defined above.

In an alternative embodiment, the method for carrying out a biospecific binding assay comprises an additional step of drying the biocomplex. In a preferred embodiment, the drying step occurs after step a) and before step b).

### A solid support

Still another aspect of the invention relates to a solid support material conjugated with a luminescent lanthanide chelate as defined hereinabove. The luminescent lanthanide chelate is typically immobilized to the solid support material either covalently or non-covalently.

In some interesting embodiments, the solid support material is selected from a nano-particle, a microparticle, a slide, a plate, and a solid phase synthesis resin.

The novel lanthanide chelates ligands and the corresponding luminescent lanthanide chelates and labelled biospecific binding reactant are based on a cyclic ligand structure which provides surprisingly efficiently excitation of the chelated lanthanide ion. At the same time, all important features of the luminescent lanthanide chelate and labelled biospecific binding reactant can be retained without any additional formation of aggregates and purification problems.

The chelates of the present invention aim to combine several important features in a single label such as:
(a) Broad excitation wavelengths at around 350 nm (see the Examples) enables the use of UV LEDs as an excitation source which will provide a cost reduction in instrument manufacturing, and the possibility of instrument miniaturization.
(b) The chelates are applicable to different lanthanides.
(c) The high luminescence of the ligands means that it is possible to decrease the labeling degree without loss of signal.
(d) The lower degree of labeling can improve the affinity of the biomolecule and decrease unspecific binding during the assay. Thus faster kinetic is possible and lower background is seen which can also improve the assay sensitivity.
(e) Reduction of unwanted adsorption properties of the chromophore moiety with improved aqueous solubility, especially concerning chelates with several aromatic chromophore moieties. This should reduce the unspecific binding of the labeled antibody and give improved assay sensitivity.
(f) Improved stability of the chelate means that more demanding assay conditions can be used such as high temperature, long incubation times and high concentrations of additional metal ions.

### EXPERIMENTAL SECTION

### Examples

The following non-limiting examples are aimed to further demonstrate the invention. The structures and synthetic routes employed are presented in Schemes 1-5.
¹H-NMR spectra were recorded with Bruker AVANCE DRX 500 MHz. Tetramethyl silane was used as internal reference. Mass spectra were recorded PerSeptive Biosystems Voyager DE-PRO MALDI-TOF instrument using α-cyano-4-cinnamic acid matrix. UV-Vis spectra were recorded on Pharmacia Ultrospec 3300 pro. Fluorescence efficiencies were determined with Perkin-Elmer Wallac Victor platefluorometer. Eu-content of Eu-chelates and labelled antibodies were measured by using ICP-MS instrument, PerkinElmer 6100 DRC Plus, in quantitative mode. The excitation, emission spectra and decay times were recorded on a Varian Cary Eclipse fluorescence spectrometer.

Conditions for HPLC purification runs: Reversed phase HPLC (RP-18 column). The solvents were A: triethyl ammonium acetate buffer (20mM, pH7) and B: 50% acetonitrile in triethyl ammonium acetate buffer (20mM, pH7). The gradient was started from 5% of solvent B and the amount of solvent B was linearly raised to 100 % in 30 minutes. Column chromatography was performed with columns packed with silica gel 60 (Merck). FC = Flash chromatography, RT = room temperature.

### Example 1. Synthesis of compound 3

A mixture of the compound **1** (0.34 g, 1.60 mmol; WO2011026790) and **2** (0.47 g, 2.15 mmol; Takalo, H., et al., Helv. Chim. Acta, 79(1996)789) in dry TEA (5 ml) and THF (10 ml) was de-aerated with argon. After addition of bis(triphenylphosphine)palladium(II) chloride (19 mg, 27 µmol) and Cul (10 mg, 53 µmol), the mixture was stirred for 24 hours at 55°C. After evaporation to dryness, the product (0.49 g, 94%) was purified by FC (silica gel, 10% EtOH/DCM/1% TEA). ¹H-NMR (CDCl₃): 8.49 (1H, s), 8.08 (1H, s), 7.66 (2H, d, J=8.7 Hz), 7.60 (1H, s,), 7.59 (2H, d, J=8.7 Hz), 4.85 (2H, s), 4.49 (2 H, q, J=7.1 Hz), 1.43 (3H, t, J=7.1 Hz). ¹³C-NMR (CDCl₃): 164.55, 160.42, 155.24, 154.94, 154.64, 154.34, 147.42, 136.24, 133.10, 132.99, 125.58, 125.27, 120.30, 119.36, 118.94, 116.65, 114.35, 112.06, 94.24, 87.57, 64.30, 62.10, 14.18. MALDI TOF-MS mass: calculated (M+H⁺) 393.18; found 394.16

### Example 2. Synthesis of compound 4

A mixture of compound **3** (0.47 g, 1.20 mmol) and PBr₃ (0.17 ml, 1.80 mmol) in dry CHCl₃ (40 ml) was stirred for 18 h at +55 °C, neutralized with 5% NaHCO₃ solution (20 ml), the aqueous phase was extracted with CHCl₃ (2x10 ml) and the combined organic phases were dried with Na₂SO₄. The product (0.43 g, 78%) was purified by FC (silica gel, 10% EtOH/DCM). ¹H-NMR (CDCl₃): 8.25 (1H, s), 8.01 (1H, d, J=1.1 Hz), 7.75 (1H, d, J=1.1 Hz); 7.68 (2H, d, J=8.7 Hz), 7.65 (2H, d, J=8.7 Hz); 4.62 (2H, s), 4.50 (2H, q, J=7.1 Hz), 1.45 (3H, t, J=7.1 Hz). ¹³C-NMR (CDCl₃): 164.32, 157.68, 155.16, 154.85, 154.55, 154.25, 148.06, 136.21, 133.59, 133.06, 128.36, 126.09, 120.26, 119.32, 118.92, 116.62, 114.32, 112.03, 94.61, 86.29, 62.25, 32.62, 14.20. MALDI TOF-MS mass: calculated (M+H⁺) 455.02 and 457.02; found 455.78 and 457.73.

### Example 3. Synthesis of compound 6

A mixture of compound **4** (0.41 g, 0.90 mmol), **5** (0.14 g, 0.82 mmol), dry K₂CO₃ (0.23 g, 1.62 mmol) and dry MeCN (8 ml) was stirred for 24 h at RT. After filtration and washing the solid material with DCM, the filtrate was evaporated to dryness. The product (0.31 g, 53%) was purified by FC (silica gel, from 1% to 3% EtOH/DCM). ¹H-NMR (D₆-DMSO): 11.48 (1H,s), 7.97 (1 H, s), 7.78-7.85 (3H, m), 7.66 (2H, d, J=8.3 Hz), 4.38 (2H, q, J=7.1 Hz); 3.80-3.85 (2H, m), 3.10-3.45 (8H, m), 2.65-2.75 (2H, m), 2.65-2.55 (2H, m), 1.43 (3H, s), 1.42 (3H, s), 1.40 (6H, s), 1.39 (6H, s), 1.34 (3 H, t, J=7.1 Hz). ¹³C-NMR (D₆-DMSO): 164.09, 155.78, 154.96, 154.80, 154.70, 154.56, 154.37, 154.08, 147.22, 137.51, 132.74, 132. 54, 129.33, 127.03, 124.69, 120.85, 118.97, 116.69, 114.39, 112.62, 93.89, 86.35, 78.71, 61.44, 61.29, 51.42, 50.18, 49,69, 28.03, 14.02. Both spectra indicate the existence of rigid compound having different structural isomers. MALDI TOF-MS mass: calculated (M+H⁺) 704.33; found 705.09.

### Example 4. Synthesis of compound 7

A mixture of compound **6** (0.29 g, 0.41 mmol) and TFA (2 ml) was stirred for 2 h at RT, evaporated to dryness and triturated with Et₂O (40 ml). The product (0.34 g, 89%) was centrifuged, washed with Et₂O (2x15 ml) and dried. ¹H NMR (D₆-DMSO): 11.54 (1H, s), 7.82 (2 H, d, J=8.4 Hz), 7.81 (1 H, s), 7.80 (1H, s), 7.71 (2 H, d, J=8.4 Hz), 4.44 (2H, q, J=7.0 Hz), 4.17 (2H, s), 3.69 (4H, bs), 3,26 (4H, bs), 2.97 (4H, bs), 1.39 (3H, t, J=7.0 Hz). ¹³C NMR (D₆-DMSO): 154.41, 160.60, 155.48, 155.18, 154.89, 154.59, 147.17, 138.30, 133.24, 133.06, 129.87, 128.40, 125.22, 121.41, 118,57, 116.19, 114.84, 112.54, 95.54, 86.36, 62.47, 57.68, 50.42, 45.90, 45.36, 14,45. MALDI TOF-MS mass: calculated (M+2H⁺) 505.54; found 505.31.

### Example 5. Synthesis of compound 10

This compound **10** was synthesized from the compound **8** (WO2013026790) and **2** using a method analogous to the synthesis described in the Example 1. Yield: 76%. ¹H-NMR (CDCl₃): 8.11 (1H, d, J=0.5 Hz), 7.64 (1H, d, J=0.5 Hz), 6.09 (2H, s), 4.84 (2 H, s), 4.70 (4H, s), 4.58 (2H, s), 4.47 (2H, q, J=7.1 Hz), 4.29 (4H, q, J=7.1 Hz), 4.28 (2 H, q, J=7.1 Hz), 3.45 (1 H, bs), 1.44 (3H, t, J=7.1 Hz), 1.32 (3H, t, J=7.1 Hz), 1.31 (6H, t, J=7.1 Hz). ¹³C-NMR (CDCl₃): 167.97, 167.89, 164.70, 161.04, 160.22, 158.87, 147.21, 134.13, 125.42, 125.17, 96.17, 94.27, 93.80, 87.78, 66.21, 65.42, 64.29, 61.86, 61.61, 61.49, 14.20, 14.08, 14.06. MALDI TOF-MS mass: calculated (M+H⁺) 588.56; found 589.03.

### Example 6. Synthesis of compound 11

This compound **11** was synthesized from the compound **9** (WO2013092992) and **2** using a method analogous to the synthesis described in the Example 1. Yield: 80%. ¹H-NMR (CDCl₃): 8.08 (2H, s), 7.60 (2H, s), 7.45 (1H, d J=8.5 Hz), 6.50 (1H, dd, J=2.0 and 8.5 Hz), 6.45 (1H, d, J=2.0 Hz), 4.85 (2H, s), 4.71 (2H, s), 4.63 (2H, s), 4.47 (2H, q, J=7.1 Hz), 4.30 (2H, q, J=7.1 Hz), 4.29 (2H, q, J=7.1 Hz), 1.44 (3H, t, J=7.1 Hz), 1.32 (3H, t, J=7.1 Hz), 1.31 (3H, t, J=7.1 Hz). ¹³C-NMR (CDCl₃): 168.11, 168.00, 164.63, 161.71, 160.08, 159.95, 147.27, 134.81, 127.02, 125.50, 106.42, 105.38, 100.99, 91.30, 89.67, 65.91, 65.34, 64.32, 62.24, 61.93, 61.54, 14.20, 14.19, 14.07. MALDI TOF-MS mass: calculated (M+H⁺) 486.18; found 486.46.

### Example 7. Synthesis of compound 12

A mixture of compound **10** (0.36 g, 0.61 mmol) and PBr₃ (86 µl, 0.92 mmol) in dry CHCl₃ (20 ml) was stirred for 2.5 h at RT, neutralized with 5% NaHCO₃ solution (20 ml), the aqueous phase was extracted with CHCl₃ (20 ml) and the combined organic phases were dried with Na₂SO₄. The product (0.33 g, 82%) was purified by FC (silica gel, 10% EtOH/DCM). ¹H-NMR (CDCl₃): 8.12 (1H, d, J=1.3 Hz), 7.79 (1H, d, J=1.3 Hz), 6.08 (2H, s), 4.71 (4H, s), 4.59 (2H, s), 4.51 (2H, s), 4.48 (2H, q, J=7.1 Hz), 4.30 (4H, q, J=7.1 Hz), 4.29 (2H, q, J=7.1 Hz), 1.43 (3H, t, J=7.1 Hz), 1.31 (9 H, t, J=7.1 Hz). ¹³C-NMR (CDCl₃): 167.92, 167.79, 164.52, 156.29, 133.27, 125.98, 125.08, 96.09, 93.92, 93.76, 88.21, 66.22, 65.59, 65.43, 62.04, 61.62, 61.51, 32.96, 14.22, 14.07, 14.03. MALDI TOF-MS mass: calculated (M+H⁺) 650.13 and 652.13; found 651.08 and 653.02.

### Example 8. Synthesis of compound 13

This compound **13** was synthesized from the compound **11** using a method analogous to the synthesis described in the Example 7. Yield: 89%. ¹H-NMR (CDCl₃): 8.10 (1H, d, J=1.2 Hz), 7.75 (1H, d, J=1.2 Hz), 7.46 (1 H, d, J=8.5 Hz), 6.50 (1H, dd, J=2.3 and 8.5 Hz), 6.46 (1H, d, J=2.3 Hz), 4.72 (2H, s), 4.63 (2H, s), 4.60 (2H, s), 4.49 (2H, q, J=7.1 Hz), 4.30 (2H, q, J=7.1 Hz), 4.29 (2H, q, J=7.1 Hz), 1.45 (3H, t, J=7.1 Hz), 1.32 (3H, t, J=7.1 Hz), 1.31 (3H, t, J=7.1 Hz). ¹³C-NMR (CDCl₃): 168.10, 167.97, 164.43, 160.05, 160.03, 157.41, 147.95, 134.88, 127.65, 126.00, 106.42, 105.28, 100.97, 91.97, 89.34, 65.92, 65.35, 62.40, 62.09, 61.54, 61.49, 32.88, 14. 22, 14.09, 14.97. MALDI TOF-MS mass: calculated (M+H⁺) 548.09 and 550.09; found 548.83 and 550.80.

### Example 9. Synthesis of compound 14

A mixture of compound **7** (0.12 g, 0.15 mmol), **12** (0.21 g, 0.32 g), DIPEA (0.4 ml) and dry MeCN (3 ml) was stirred for 5.5 h at RT and evaporated to dryness. The product (0.19 g, 79%) was purified by FC (silica gel, first from 10% EtOH/DCM to 15% EtOH/DCM, then 15% EtOH/DCM/5% TEA). As the product contains 2-3 rigid isomers, the NMR spectra were too complicated to assigned the isomers. MALDI TOF-MS mass: calculated (M+H⁺) 1642.60; found 1643.57.

### Example 10. Synthesis of compound 15

This compound **15** was synthesized from the compound **13** using a method analogous to the synthesis described in the Example 9. The product was purified by FC (silica gel, from 2% EtOH/DCM/1% TEA). Yield: 84%. As the product contains 2-3 rigid isomers, the NMR spectra were too complicated to assigned the isomers. MALDI TOF-MS mass: calculated (M+H⁺) 1438.54; found 1439.41.

### Example 11. Synthesis of compound 16

A mixture of the compound **14** (92 mg, 64 µmol) and 0.5M KOH in EtOH (6.5 ml) was stirred for 1 h at RT and H₂O (3 ml) was added. After stirring for 4 hours at RT, EtOH was evaporated, some H₂O (2ml) added and the residue was stirred for 24 h at RT. After addition of citric acid (41 mg, 0.21 mmol) in H₂O (0.25 ml), the pH was adjusted to ca. 6.5 with 6M HCI. Europium(III) chloride (26 mg, 71 µmol) in H₂O (0.25 ml) was added within 10 minutes and the pH was adjusted to ca. 9.5 with 1M NaOH. The mixture was stirred for 4-6 weeks at 95 °C (after the analytical HPLC chromatogram showed completed complexation), the pH was adjusted to ca. 7.0 with 1M HCI, evaporated to dryness, dissolved in 20 mmol TEAA buffer (1 ml) and purified with semi-preparative HPLC. R_{f}(HPLC)=16.0 min. UV=360nm. MALDI TOF-MS mass: calculated (M+6H⁺) 1443.23; found 1443.96.

Ligand isomers shown in HPLC during Eu(III)-loading: 1) R_{f}(HPLC)=18.5 min, UV=345 nm; 2) R_{f}(HPLC)=20.4 min, UV=347 nm; 3) R_{f}(HPLC)=21.7 min, UV=347 nm. All this peaks finally gave the product peak at R_{f} =16.0 min. and UV=360 nm. The Eu complex formation caused the observed bathochromic shift of 13-15 nm at UV. This was separately secured by additional HPLC purification of the ligand isomers and loading of Eu(III) ion to each isomers. All those loadings gave finally the same product at R_{f}(HPLC)=16.0 min.

It is noted herein that the general Eu-loading methods disclosed in literature, patents and patents applications with similar macrocyclic ligands did not give the wanted chelates and only non-complexed ligands were obtained. After extensive experimentation it was determined that Eu loading required high pH (>9), high temperatures of around 80-90°C, and long incubation times of around two weeks. The difficulty of loading the Eu is indicating the high chelating stability once the chelates are formed.

### Example 12. Synthesis of compound 17

This compound **17** was synthesized from the compound **15** using a method analogous to the synthesis described in the Example 11. R_{f}(HPLC)=19.2 min. UV=350nm. MALDI TOF-MS mass: calculated (M+4H⁺) 1295.23; found 1295.85.

Ligand isomers shown in the HPLC during Eu(III) loading: 1) R_{f}(HPLC)=21.7 min, UV=347 nm; 2) R_{f}(HPLC)=22.3 min, UV=339 nm; 3) R_{f}(HPLC)=23.6 min, UV=343 nm.

### Example 13. Synthesis of compound 18

Compound **16** (43 mg, 21 µmol) in H₂O (1 ml) was added within 5 min to a mixture of CSCl₂ (22 µl, 0.29 mmol) and NaHCO₃ (28 mg, 0.33 mmol) and CHCl₃ (1 ml). After stirring for 40 min at RT, the aqueous phase was washed with CHCl₃ (3x1 ml). The product was precipitated with acetone, centrifuged and washed with acetone.

### Example 14. Synthesis of compound 19

This compound **19** was synthesized from the compound **17** using a method analogous to the synthesis described in the Example 13.

### Example 15. Synthesis of compound 20

A mixture of compound **18** (2 mg) and taurine (2 mg) in 50 mM Na₂CO₃ buffer (300 µl, pH 9.8) was stirred for o/n at RT. The product was purified by using semi-preparative HPLC. R_{f}(HPLC)=15.2 min. UV=354 nm.

After the product fractions were evaporated and the residue was dissolved in 50 mM TRIS buffer (1 ml). The Eu concentration was measured by ICP-MS. The analyzing parameters were: the Peak Hopping mode, 20 sweeps/reading, 7 replicates, the Dwell time and the integration time was 50 ms and 1000 ms, respectively. Rhodium was used as the internal standard and the Europium was measured on Mass 152.929. A commercial multi-standard from Ultra Scientific, IMS-101, ICP-MS calibration standard 1 was used for the calibration.

The sample preparation for the ICP-MS was done by using a digestion procedure i.e. a microwave digestion system from Anton Paar, Microwave Sample preparation System, Multiwave 3000. The Eu chelate in the 50 mM TRIS buffer was digested with microwave in mixture of Suprapur acids, HNO₃ (5 ml) and H₂O₂ (1 ml). Afterwards the sample was diluted with deionized water (100 ml).

### Example 16. Synthesis of compound 21

This compound **21** was synthesized from the compound **19** using a method analogous to the synthesis described in the Example 15. R_{f}(HPLC)=17.2 min. UV=348 nm.

After the product fractions were evaporated and the residue was dissolved in 50 mM TRIS buffer (1 ml). The Eu concentration was measured by IPC-MS using a method analogous in the Example 15.

### Example 17. Labeling of antibody with labelling reagents 18 and 19

Labeling of an Tnl antibody was performed as described in von Lode P. et al., Anal. Chem., 2003, 75, 3193-3201 by using 300 fold excess of the labelling reagents **18** or **19.** The reactions were carried out overnight at RT. Labeled antibody was separated from the excess of chelates on Superdex 200 HR 10/30 gel filtration column (GE healthcare) by using Tris-saline-azide buffer (Tris 50 mM, NaCl 0.9%, pH 7.75) as an eluent. The fractions containing the antibody were pooled and the Eu concentration was measured by UV and secured by IPC-MS described in the Example 15.

### Example 18. Troponin I immunoassay

The Tnl antibody labeled with the chelate **18** or **19** was tested in sandwich immunoassay for cardiac troponin I. As a reference compound a Tnl antibody labelled with α-gal-9-D Eu (von Lode P. et al., Anal. Chem., 2003, 75, 3193-3201) was used. 10 µl of diluted tracer antibody (5ng/µl) and 20 µl of Tnl standard solution were pipetted to a pre-coated assay well (single wells in 96 well plate format, wells coated with streptavidin and a biotinylated capture antibody against Tnl, Innotrac Diagnostics). The reaction mixtures were incubated 20 min at 36°C with shaking. The wells were washed 6 times and dried prior to measurement with Victor™ Plate fluorometer.

The conventional 9-dentate α-galactose Eu chelate (Ref in Table 1) was prepared according to von Lode P. et al., Anal. Chem., 2003, 75, 3193-3201.

The results are summarized in Table 1. Both A and B standards were measured in 12 replicates and other standards C-F in 6 replicates.

**Table 1.**

| **Compound** | **AS** | **Std A** | **Std B1** | **Std B2** | **Std B** | **Std C** | **Std D** | **Std E** | **Std F** | |
|---|---|---|---|---|---|---|---|---|---|---|
| | | **0** | **0,004** | **0,0085** | **0,04** | **0,11** | **0,94** | **7,1** | **63,9** | **Eu/IgG** |
| Ref | 239 | 243 | 61 | 113 | 363 | 1 235 | 10 043 | 73 495 | 544 110 | 11.4 |
| **18** | 2 375 | 2 694 | 91 | 359 | 1 350 | 5 665 | 42 910 | 334 944 | 2 569 003 | 6.2 |
| **19** | 2 934 | 2 787 | 45 | 304 | 1 535 | 4 995 | 43 539 | 340 379 | 2 683 694 | 11.2 |

### Example 19. Photo-physical properties of novel chelates conjugated to taurine (chelates 20 and 21) and the labelled cTnl antibodies with chelates 18 and 19.

The measured photo-physical properties excitation wavelengths (λ_{exc}), luminescence decay times (τ), molar absorptivities (ε), estimated luminescence yields (εΦ) of the novel chelates (**20** and **21**) and the labelled cTnl antibodies with chelates **18** and **19** in 50 mM TRIS buffer (pH 7.75) are in the Table 2.

Dry measurements (**18** (dry) and **19** (dry)) represents estimated luminescence yields based on the signal measurements after dry immunoassay done as described in the Example 18.

**Table 2.**

| Compound | λ_{exc}/nm | τ/ms | ε/M⁻¹cm⁻¹ | εΦ/M⁻¹cm⁻¹ |
|---|---|---|---|---|
| **20** | 345 | 0,48 | 77000 | 700 |
| **21** | 341 | 0,58 | 89000 | 600 |
| **18^{a)}** | 348 | 0,42 | 104000 | 1000 |
| **18^{a)}** (dry) | | | | **69 500** |
| **19^{a)}** | 346 | 0,51 | 99000 | 500 |
| **19^{a)}** (dry) | | | | **40 200** |

| | | | | |
|---|---|---|---|---|
| ^{a}Coupled to protein | | | | |

As it can be seen from the results in the Table 2, the taurine derivatives and labeled IgG has rather low luminescence (below 1000 M⁻¹cm⁻¹) when measured in aqueous buffer, but the signals are much enhanced in dry format i.e. approximately 80-100 fold increase of brightness. This surprising improvement in luminescence in the dry format means that the skilled person can significantly improve the sensitivity of the assay - if necessary - by simply adding a drying step.

Without wishing to be bound by theory, it is hypothesised that the low luminescence intensities in aqueous buffer can be explained by the low-lying CT-state of the ligand, as has been published with similar type of pyridine dicarboxylic acids (see: Andraud, C., et al. in Eur. J. Inorg. Chem 2009, 4357; Inorg. Chem. 2011, 4987 and results of Takalo, H., et al., 2010, a poster presentation in the 1st International Conference on luminescence of Lanthanides Odessa, Ukraine). Moreover, such low luminescence has not previously been shown to be enhanced so significantly in dry measurement format.

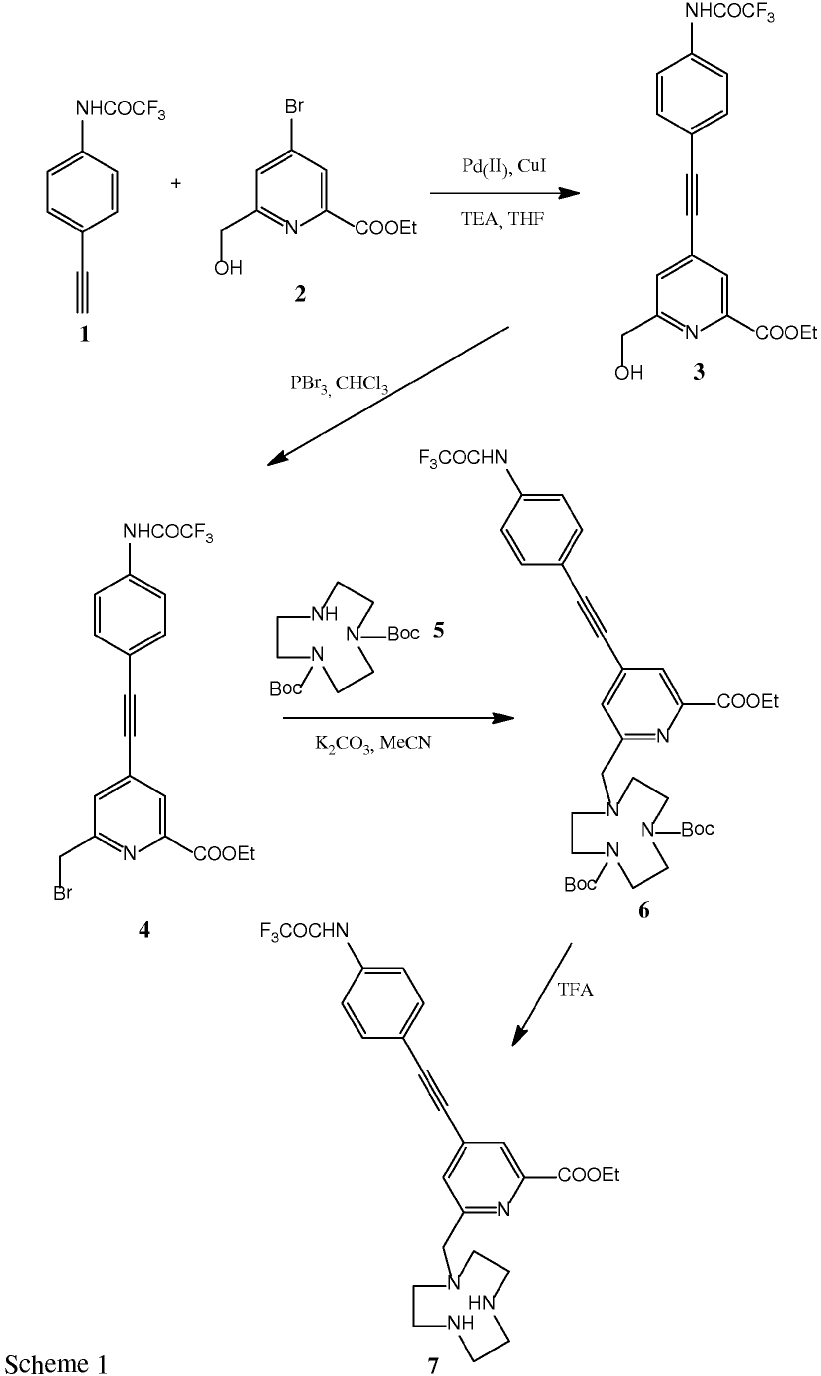

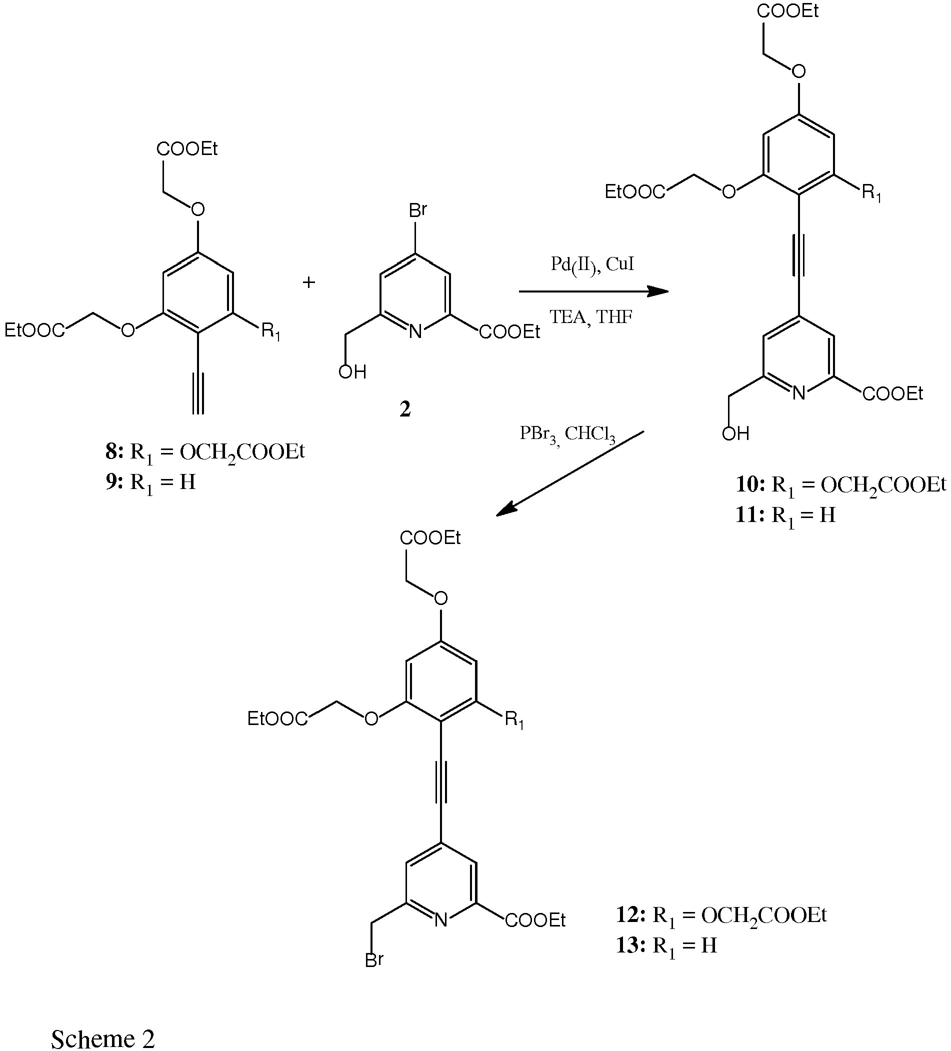

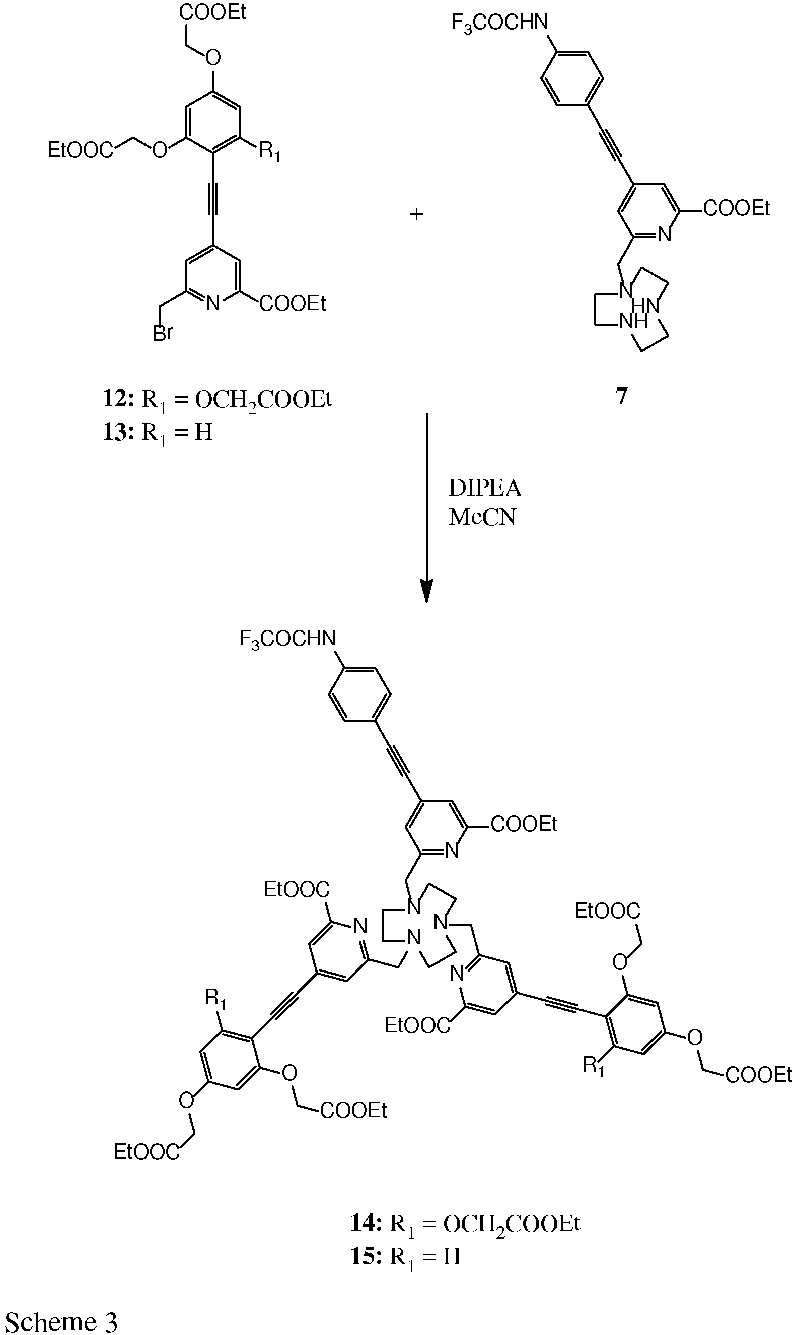

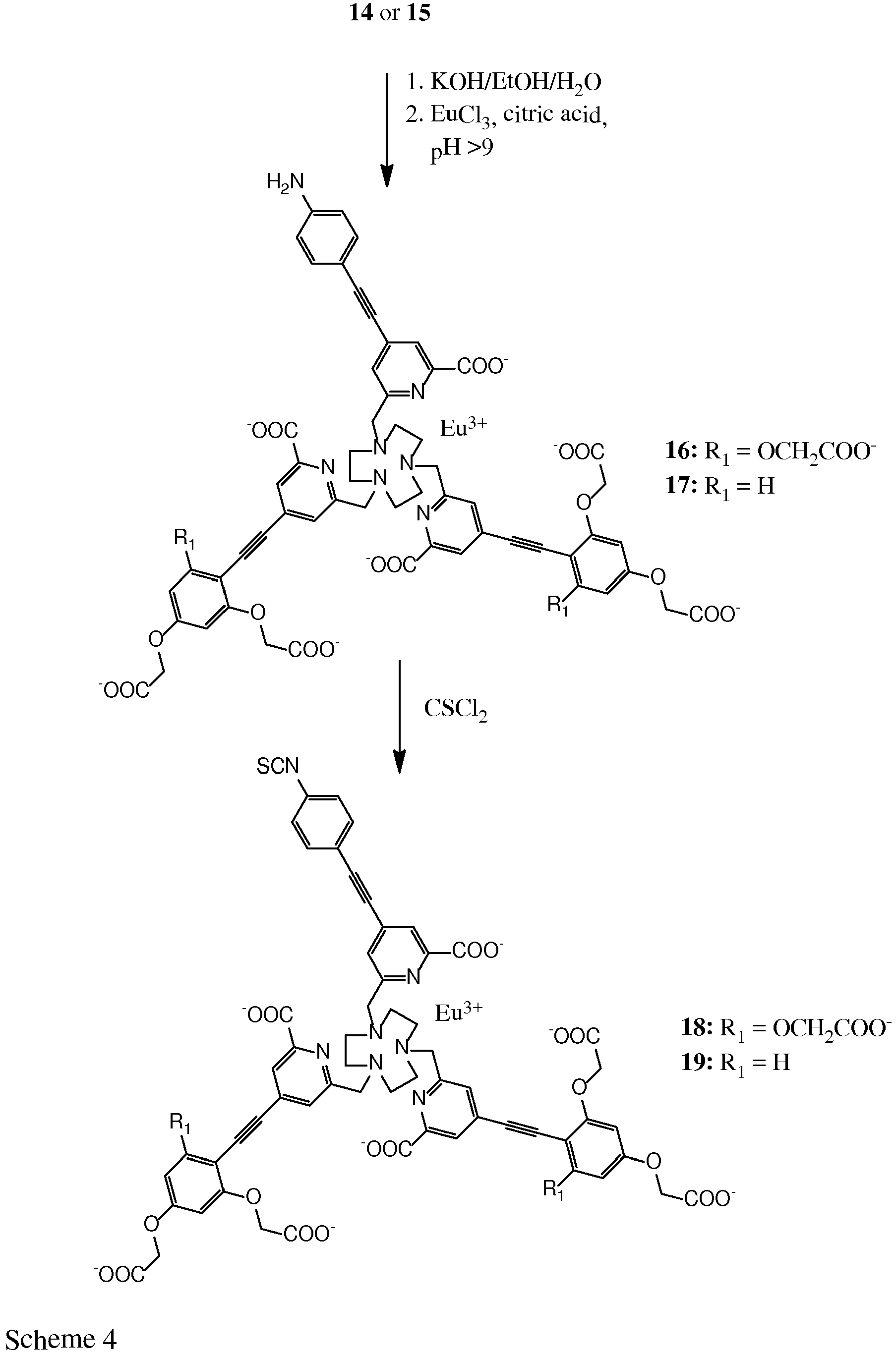

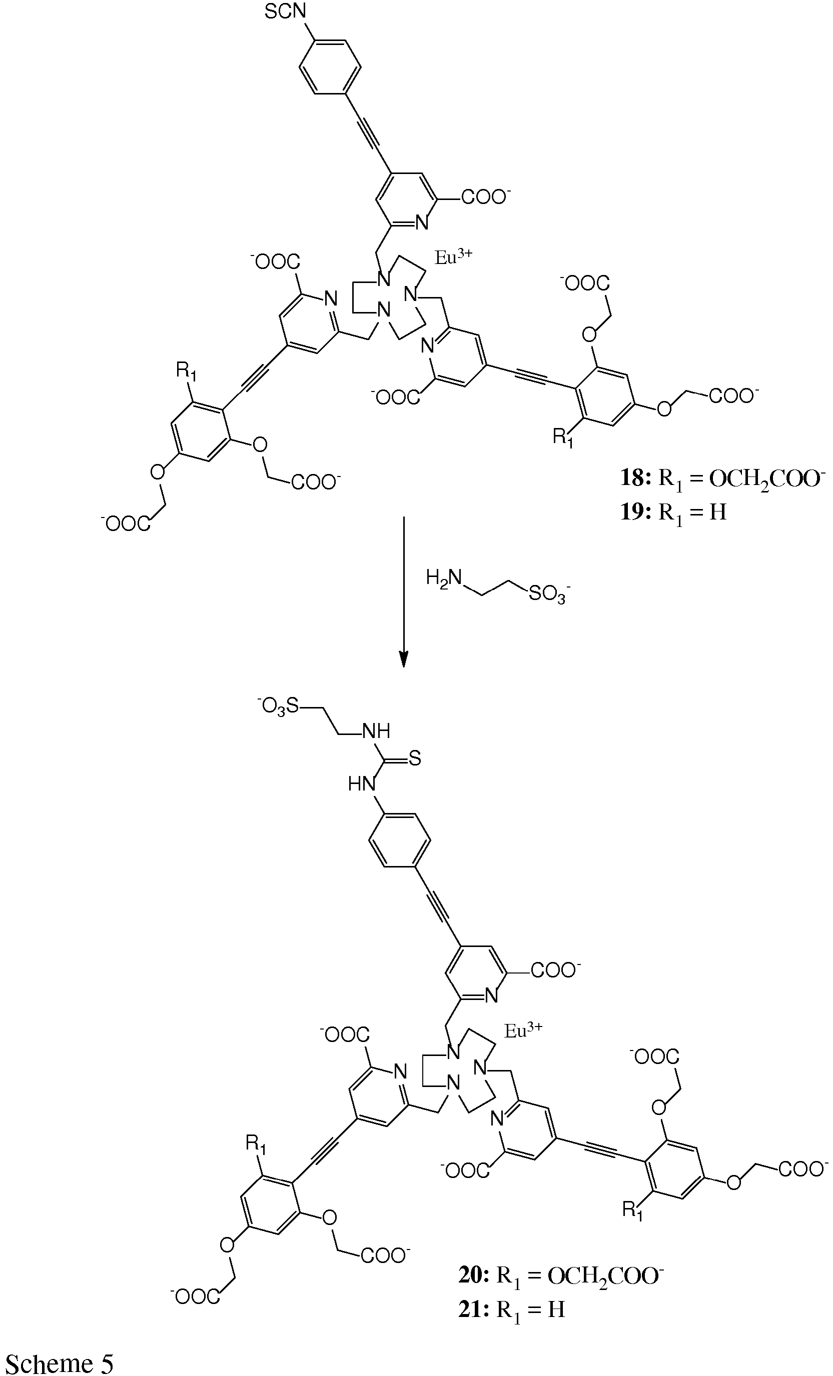

## Claims

1. A luminescent lanthanide chelate of formula (I) or a salt thereof: 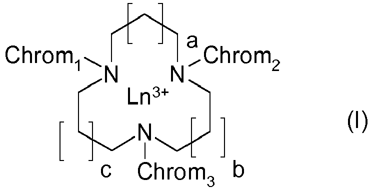 wherein a, b, and c are independently selected from 0 and 1; and
Ln³⁺ is selected from Eu^{3+,} Tb³⁺, Dy³⁺, and Sm³⁺; and
Chrom₁, Chrom₂, and Chrom₃ are of formula (II): 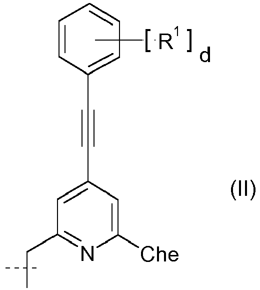
wherein Che is a chelating group independently selected from -CO₂H, -PO₃H₂, - PO(OH)R², -CH₂PO₃H₂, and -CONR³R⁴,
R² is selected from phenyl, benzyl, methyl, ethyl, propyl, n-butyl, iso-butyl, sec-butyl or tert-butyl,
R³ and R⁴ are independently selected from hydrogen and -L¹-Z¹, wherein L¹ is a direct bond or a spacer group, and Z¹ is a reactive group enabling the chelate to be linked to a biospecific reactant; and
d is 1, 2, 3, 4, or 5;
R¹ is one or more substituents independently selected from any one of the group consisting of:
(i) hydrogen,
(ii) an electron donating solubilising group selected from -X-R⁵ wherein X is an oxygen atom, a sulphur atom, or-N(R⁶)CO-, R⁶ is hydrogen or C₁₋₆alkyl, and R⁵ is selected from hydrogen, -C₁₋₆alkyl, -(CH₂)₁₋₆OH, -(CH₂)₁₋₆OC₁₋₆alkyl, -(CH₂)₁₋₆CO₂H, -(CH₂)₁₋₆CONR⁷R⁸, -(CH₂)₁₋₆SO₃H, -(CH₂)₁₋₆N(CH₃)₂, -(CH₂)₁₋₆N(CH₃)₂⁺-(CH₂)₁₋₆SO₃⁻ and polyethylene glycol, wherein R⁷ and R⁸ are each independently selected from hydrogen, C₁₋₆alkyl, C₁₋₆alkyl-OH, -CH(CH₂OH)₂, and -CH(CH₂OH)₃,
(iii) a group selected from C₁₋₆alkyl, -(CH₂)₁₋₆OH, -(CH₂)₁₋₆OCH₃, -(CH₂)₁₋₆SCH₃
(iv) -L²-Z², wherein L² is a direct bond or a spacer group, and Z² is a reactive group enabling the chelating agent to be linked to a molecule to be labelled,
wherein the spacer groups L¹ and L² have a length of 1-20 bonds between the attachment point and reactive group, and are formed of one to five moieties, each moiety selected from the group consisting of phenylene, alkylene containing 1-10 carbon atoms, an ethynediyl (-C=C-), an ether (-O-), a thioether (-S-), a disulfide (-S-S-), an amide (-C(=O)-NH-, -C(=O)-NCH₃- and -NCH₃-C(=O)-), a thiourea (-NH-C(=S)-NH-) and a triazole,
wherein Z¹ or Z² is selected from azido (-N₃), alkynyl (-C≡CH), alkylene (-CH=CH₂), amino (-NH₂), aminooxy (-O-NH₂), aldehyde (-CHO), hydrazide (-CONHNH₂), mercapto (-SH), maleimido, activated derivatives of maleimido, isocyanato (-NCO), isothiocyanato (-NCS), diazonium (-N⁺N), bromoacetamido, iodoacetamido, pyridyl-2-dithio, and 6-substituted 4-chloro-1,3,5-triazin-2-ylamino,
wherein two of the groups Chrom₁, Chrom₂, and Chrom₃ have two or three R¹ substituents selected from group (ii) in the para and ortho positions in relation to the acetylene group;
wherein the third Chrom group is substituted with -L²-Z².

2. The chelate according to claim 1 wherein a = b = c = 0.

3. The chelate according to claim 1 or claim 2 wherein at least one of the groups Chrom₁, Chrom₂, and Chrom₃ is selected from formula (IIa), (IIb) or (IIc): 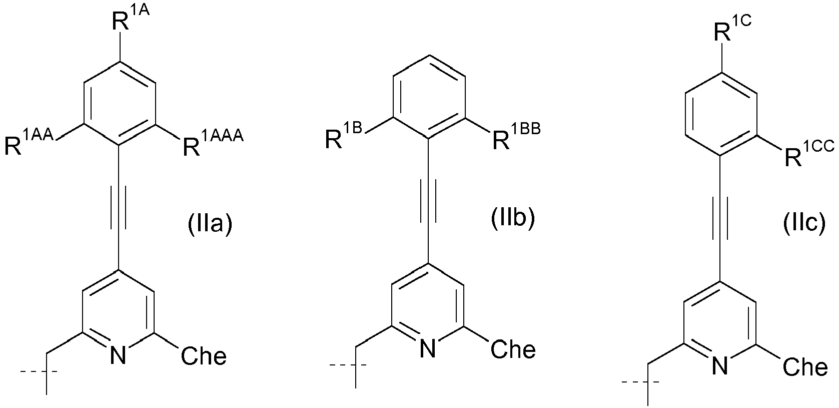 wherein R^{1A}, R^{1AA}, R^{1AAA}, R^{1B}, R^{1BB}, R^{1C}, and R^{1CC} are each independently selected from R¹ group (ii) as set out in claim 1.

4. The chelate according to any one of claims 1 to 3 wherein at least two of the groups Chrom₁, Chrom₂, and Chrom₃ are selected from formula (IIa), (IIb) or (IIc) as defined in claim 3.

5. The chelate according to any one of claims 1 to 4 wherein one of the groups Chrom₁, Chrom₂, and Chrom₃ is selected from (IId), (IIe), (IIf) or (IIg): 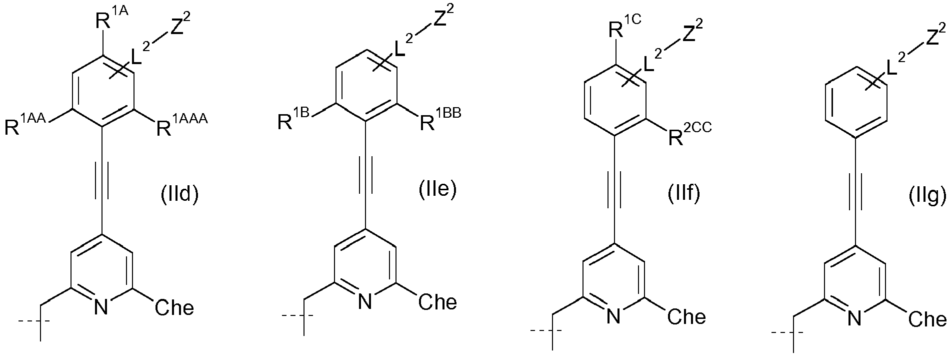 wherein R^{1A}, R^{1AA}, R^{1AAA}, R^{1B}, R^{1BB}, R^{1C}, and R^{1CC} are each independently selected from R¹ group (ii) as set out in claim 1.

6. The chelate according to any one of claims 1 to 5 wherein X = -O-.

7. The chelate according to any one of claims 1 to 6 wherein the groups L¹ or L², when present, are direct bonds.

8. The chelate according to any one of claims 1 to 7 wherein L¹ is a spacer group selected from -(CH₂)₁₋₆-, and *-(CH₂)₁₋₆Ph, wherein the bond marked * is directly connected to the nitrogen atom of -CONR³R⁴.

9. The chelate according to any one of claims 1 to 8 wherein Z¹ or Z² is an isothiocyanato (-NCS) group.

10. The chelate according to any one of claims 1 to 9 wherein L² is a direct bond and Z² is an isothiocyanato (-NCS) group.

11. The chelate according to any one of claims 1 to 10 wherein Che is -CO₂H.

12. The chelate having formula (IIIa) or a salt thereof: 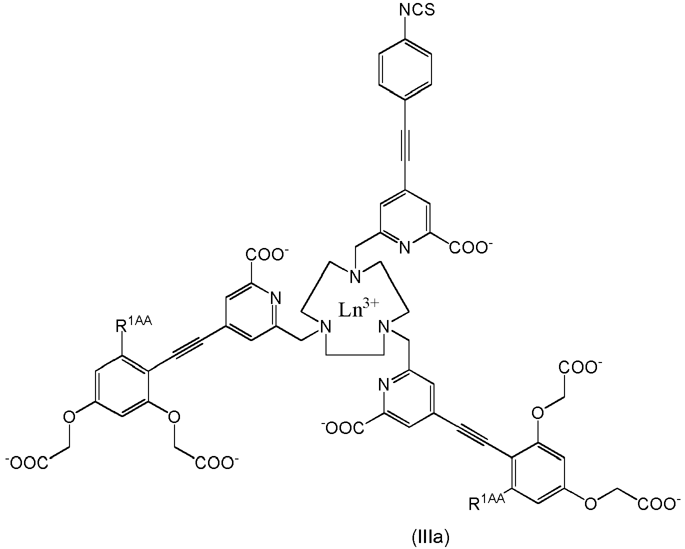 wherein R^{1AA} is hydrogen or -OCH₂CO₂⁻.

13. A detectable molecule comprising a biospecific binding reactant conjugated to a luminescent lanthanide chelate of formula (I) as defined in any one of claims 1 to 12.

14. The detectable molecule according to claim 13, wherein the biospecific binding reactant is selected from an antibody, an antigen, a receptor ligand, a specific binding protein, a DNA probe, a RNA probe, an oligopeptide, an oligonucleotide, a modified oligonucleotide, a modified polynucleotide, a protein, an oligosaccaride, a polysaccharide, a phospholipid, a PNA, a steroid, a hapten, a drug, a receptor binding ligand, and lectine.

15. The detectable molecule according to claim 13 or 14, wherein the biospecific binding reactant is an antibody.

16. A lanthanide chelating ligand of formula (IV) or a salt thereof 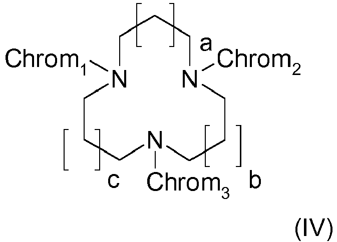 wherein a, b, c, Chrom₁, Chrom₂, and Chrom₃ are as defined in any one of claims 1 to 12.

17. A method of carrying out a biospecific binding assay, said method comprising the steps of:
a) forming a biocomplex between an analyte and a biospecific binding reactant labelled with a luminescent lanthanide chelate according to any one of claims 1-12;
b) exciting said biocomplex with radiation having an excitation wavelength, thereby forming an excited biocomplex; and
c) detecting emission radiation emitted from said excited biocomplex.

18. Use of a detectable molecule according to any one of claims 13-15 in a specific bioaffinity based binding assay utilizing time-resolved fluorometric determination of a specific luminescence based on one or two photon excitation.

19. A solid support material conjugated with a luminescent lanthanide chelate according to any of the claims 1-12 or a lanthanide chelating ligand according to claim 16.

## Patentansprüche

1. Lumineszierendes Lanthanidchelat der Formel (I) oder Salz davon: 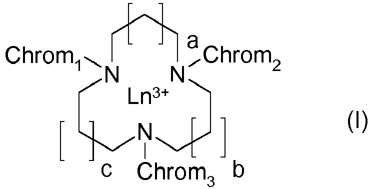 worin a, b und c unabhängig ausgewählt sind aus 0 und 1; und
Ln³⁺ ausgewählt ist aus Eu³⁺, Tb³⁺, Dy³⁺ und Sm³⁺; und
Chrom₁, Chrom₂ und Chrom₃ der Formel (II) entsprechen: 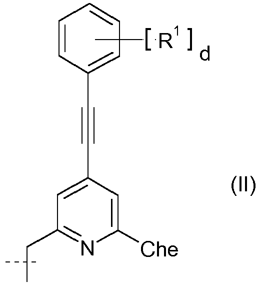
worin Che eine Chelatgruppe darstellt, die unabhängig ausgewählt ist aus -CO₂H, -PO₃H₂, -PO(OH)R², -CH₂PO₃H₂ und -CONR³R⁴,
R² ausgewählt ist aus Phenyl, Benzyl, Methyl, Ethyl, Propyl, n-Butyl, Isobutyl, sec-Butyl oder tert-Butyl,
R3 und R4 unabhängig ausgewählt sind aus Wasserstoff und -L1-Z1, worin L1 eine Direktbindung oder eine Spacergruppe und Z1 eine reaktive Gruppe darstellt, durch welche das Chelat an einen biospezifischen Reaktanden gebunden werden kann; und
d 1, 2, 3, 4 oder 5 ist;
R¹ einen oder mehrere Substituenten darstellt, unabhängig ausgewählt aus einem Element der Gruppe, bestehend aus:
(i) Wasserstoff,
(ii) einer elektronenspendenden Solubilisierungsgruppe, ausgewählt aus -X-R⁵, worin X ein Sauerstoffatom, ein Schwefelatom oder -N(R⁶)CO- ist, R⁶ Wasserstoff oder C₁₋₆-Alkyl ist, und R⁵ ausgewählt ist aus Wasserstoff, -C₁₋₆-Alkyl, -(CH₂)₁₋₆OH, -(CH₂)₁₋₆OC₁₋₆-Alkyl, -(CH₂)₁₋₆CO₂H, -(CH₂)₁₋₆CONR⁷R⁸, -(CH₂)₁₋₆SO₃H, -(CH₂)₁₋₆N(CH₃)₂, -(CH₂)₁₋₆N(CH₃)₂⁺-(CH₂)₁₋₆SO₃⁻ und Polyethylenglycol, worin R⁷ und R⁸ jeweils unabhängig ausgewählt sind aus Wasserstoff, C₁₋₆-Alkyl, C₁₋₆-Alkyl-OH, -CH(CH₂OH)₂ und -CH(CH₂OH)₃,
(iii) einer Gruppe, ausgewählt aus C₁₋₆-Alkyl, -(CH₂)₁₋₆OH, -(CH₂)₁₋₆OCH₃,-(CH₂)₁₋₆SCH₃
(iv) -L²-Z², worin L² eine Direktgruppe oder eine Spacergruppe ist, und Z² eine reaktive Gruppe ist, durch die das Chelierungsmittel an ein zu markierendes Molekül gebunden werden kann,
worin die Spacergruppen L¹ und L² eine Länge von 1 - 20 Bindungen zwischen dem Befestigungspunkt und der reaktiven Gruppe aufweisen und aus einer bis fünf Einheiten gebildet werden, worin jede Einheit ausgewählt ist aus der Gruppe bestehend aus Phenylen, Alkylen aus 1 - 10 Kohlenstoffatomen, einem Ethindiyl (-C=C-), einem Ether (-O-), einem Thioether (-S-), einem Disulfid (-S-S-), einem Amid (-C(=O)-NH-, -C(=O)-NCH₃- und -NCH₃-C(=O)-), einem Thioharnstoff (-NH-C(=S)-NH-) und einem Triazol,
worin Z¹ oder Z² ausgewählt ist aus Azido (-N₃), Alkynyl (-C≡CH), Alkylen (-CH=CH₂), Amino (-NH₂), Aminooxy (-O-NH₂), Aldehyd (-CHO), Hydrazid (-CONHNH₂), Mercapto (-SH), Maleimido, aktivierten Derivaten von Maleimido, Isocyanato (-NCO), Isothiocyanato (-NCS), Diazonium (-N⁺N), Bromacetamido, Iodacetamido, Pyridyl-2-dithio und 6-substituiertes 4-Chlor-1,3,5-triazin-2-ylamino,
worin zwei der Gruppen Chrom₁, Chrom₂ und Chrom₃ zwei oder drei R¹-Substituenten aufweisen, ausgewählt aus der Gruppe (ii) in den para- und ortho-Positionen in Bezug zur Acetylengruppe;
worin die dritte Chromgruppe substituiert ist mit -L²-Z².

2. Chelat nach Anspruch 1, worin a = b = c = 0 ist.

3. Chelat nach Anspruch 1 oder Anspruch 2, worin mindestens eine der Gruppen Chrom₁, Chrom₂ und Chrom₃ ausgewählt ist aus der Formel (IIa), (IIb) oder (IIc): 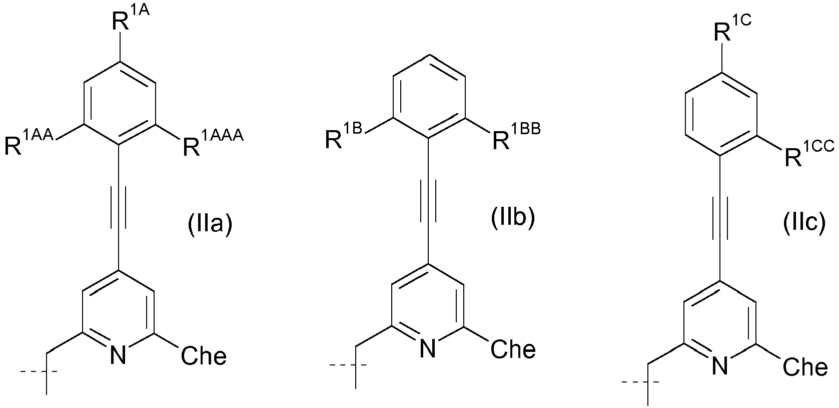 worin R^{1A}, R^{1AA}, R^{1AAA}, R^{1B}, R^{1BB}, R^{1C} und R^{1CC} jeweils unabhängig ausgewählt sind aus der R¹-Gruppe (ii), wie in Anspruch 1 festgelegt.

4. Chelat nach einem der Ansprüche 1 bis 3, worin mindestens zwei der Gruppen Chrom₁, Chrom₂ und Chrom₃ ausgewählt sind aus der Formel (IIa), (IIb) oder (IIc), wie in Anspruch 3 definiert.

5. Chelat nach einem der Ansprüche 1 bis 4, worin eine der Gruppen Chrom₁, Chrom₂ und Chrom₃ ausgewählt ist aus (IId), (IIe), (IIf) oder (IIg): 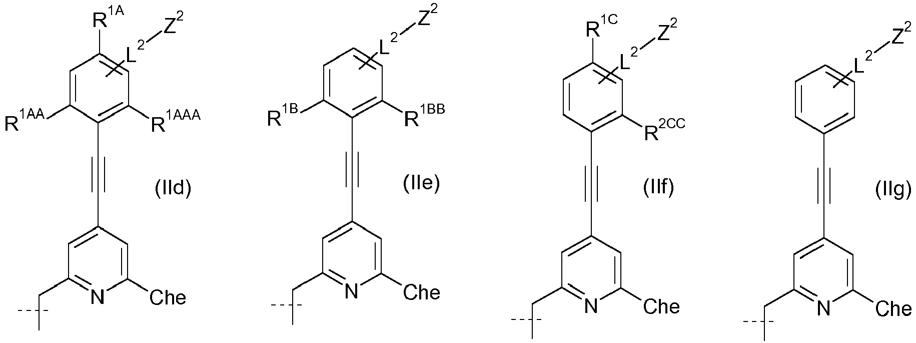 worin R^{1A}, R^{1AA}, R^{1AAA}, R^{1B}, R^{1BB}, R^{1C} und R^{1CC} jeweils unabhängig ausgewählt sind aus der R¹-Gruppe (ii), wie in Anspruch 1 festgelegt.

6. Chelat nach einem der Ansprüche 1 bis 5, worin X = -O- ist.

7. Chelat nach einem der Ansprüche 1 bis 6, worin die Gruppen L¹ oder L², falls vorhanden, Direktbindungen darstellen.

8. Chelat nach einem der Ansprüche 1 bis 7, worin L¹ eine Spacergruppe darstellt, ausgewählt aus -(CH₂)₁₋₆- und *-(CH₂)₁₋₆Ph, worin die mit * gekennzeichnete Bindung direkt mit dem Stickstoffatom von -CONR³R⁴ verbunden ist.

9. Chelat nach einem der vorhergehenden Ansprüche 1 bis 8, worin Z¹ oder Z² eine Isothiocyanato(-NCS)-Gruppe ist.

10. Chelat nach einem der Ansprüche 1 bis 9, worin L² eine Direktbindung darstellt und Z² eine Isocyanatgruppe (-NCS) darstellt.

11. Chelat nach einem der Ansprüche 1 bis 10, worin Che -CO₂H ist.

12. Chelat mit der Formel (lila) oder ein Salz davon: 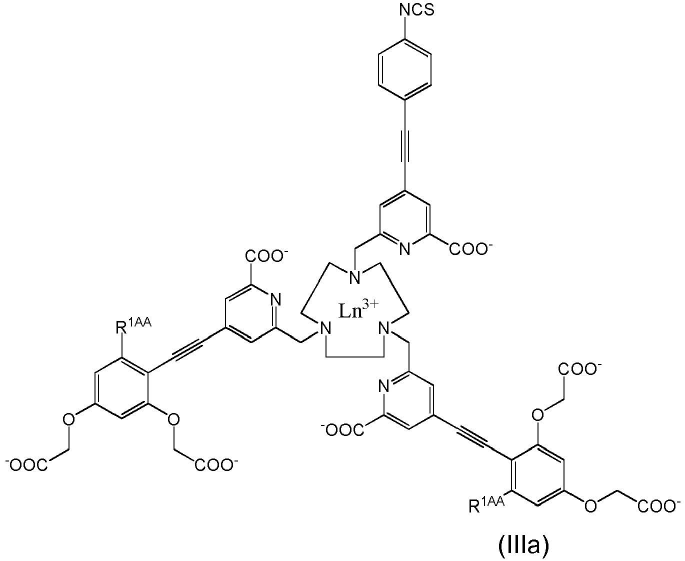 worin R^{1AA} Wasserstoff oder -OCH₂CO₂⁻ darstellt.

13. Nachweisbares Molekül, umfassend einen biospezifischen Bindungsreaktanten, das an ein lumineszierendes Lanthanidchelat der Formel (I) gebunden ist, wie in einem der vorstehenden Ansprüche 1 bis 12 definiert.

14. Nachweisbares Molekül nach Anspruch 13, wobei der biospezifische Bindungsreaktant ausgewählt ist aus einem Antikörper, einem Antigen, einem Rezeptorliganden, einem spezifischen Bindungsprotein, einer DNA-Sonde, einer RNA-Sonde, einem Oligopeptid, einem Oligonukleotid, einem modifizierten Oligonukleotid, einem modifizierten Polynukleotid, einem Protein, einem Oligosaccharid, einem Polysaccharid, einem Phospholipid, einer PNA, einem Steroid, einem Hapten, einem Arzneimittel, einem Rezeptorbindungsliganden und Lektin.

15. Nachweisbares Molekül nach Anspruch 13 oder 14, worin der biospezifische Bindungsreaktant ein Antikörper ist.

16. Lanthanidchelat-Ligand der Formel (IV) oder ein Salz davon 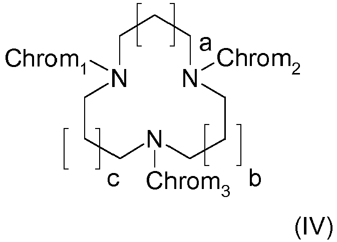 worin a, b, c, Chrom₁, Chrom₂ und Chrom₃ den Definitionen nach Anspruch 1 bis 12 entsprechen.

17. Verfahren zum Durchführen eines biospezifischen Bindungsassays, wobei das Verfahren die folgenden Schritte umfasst:
a) Bilden eines Biokomplexes zwischen dem Analyten und einem biospezifischen Reaktanten, der mit einem lumineszierenden Lanthanidchelat nach einem der Ansprüche 1 bis 12 markiert ist;
b) Anregen des Biokomplexes mit Strahlung mit einer Anregungswellenlänge, wodurch ein angeregter Biokomplex gebildet wird; und
c) Detektieren der Emissionsstrahlung, die von dem angeregten Biokomplex emittiert wird.

18. Verwendung eines nachweisbaren Moleküls nach einem der Ansprüche 13 bis 15 in einem spezifischen Bioaffinitäts-basierten Bindungsassay, der eine zeitaufgelöste fluorimetrische Bestimmung einer spezifischen Lumineszenz verwendet, die auf einer oder zwei Photonenanregungen basiert.

19. Festes Trägermaterial, konjugiert mit einem lumineszierenden Lanthanidchelat gemäß einem der Ansprüche 1 bis 12 oder mit einem Lanthanidchelatliganden nach Anspruch 16.

## Revendications

1. Chélate de lanthanide luminescent de formule (I) ou un sel de celui-ci : 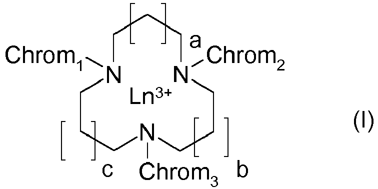 dans lequel a, b et c sont indépendamment choisis parmi 0 et 1 ; et
Ln³⁺ est choisi parmi Eu^{3+,} Tb³⁺, Dy³⁺ et Sm³⁺ ; et
Chrom₁, Chrom₂ et Chrom₃ sont de formule (II) : 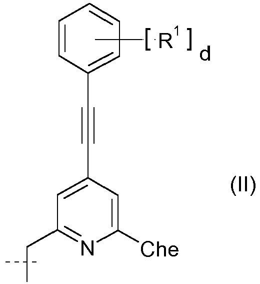
dans lequel Che est un groupe chélatant indépendamment choisi parmi -CO₂H, -PO₃H₂, -PO(OH)R², -CH₂PO₃H₂ et -CONR³R⁴,
R² est choisi parmi phényle, benzyle, méthyle, éthyle, propyle, n-butyle, iso-butyle, sec-butyle ou tert-butyle,
R³ et R⁴ sont indépendamment choisis parmi hydrogène et -L¹-Z¹, dans lequel L¹ est une liaison directe ou un groupe espaceur, et Z¹ est un groupe réactif permettant au chélate d'être lié à un réactif biospécifique ; et
d est 1, 2, 3, 4 ou 5 ;
R¹ est un ou plusieurs substituants indépendamment choisis parmi l'un quelconque du groupe constitué de :
(i) hydrogène,
(ii) un groupe solubilisant donneur d'électron choisi parmi -X-R⁵ dans lequel X est un atome d'oxygène, un atome de soufre, ou -N(R⁶)CO-, R⁶ est hydrogène ou alkyle en C_{1 à 6}, et R⁵ est choisi parmi hydrogène, -alkyle en C_{1 à 6}, -(CH₂)_{1 à 6}OH, -(CH₂)_{1 à 6}OC-alkyle en _{1 à 6}, -(CH₂)_{1 à 6}CO₂H, -(CH₂)_{1 à 6}CONR⁷R⁸, -(CH₂)_{1 à 6}SO₃H, -(CH₂)_{1 à 6}N(CH₃)₂, -(CH₂)_{1 à 6}N(CH₃)₂⁺-(CH₂)_{1 à 6}SO₃⁻ et polyéthylène glycol, dans lequel R⁷ et R⁸ sont chacun indépendamment choisis parmi hydrogène, alkyle en C_{1 à 6}, alkyle en C_{1 à 6}OH, -CH(CH₂OH)₂, et -CH(CH₂OH)₃,
(iii) un groupe choisi parmi alkyle en C_{1 à 6}, -(CH₂)_{1 à 6}OH, -(CH₂)_{1 à 6}OCH₃, -(CH₂)_{1 à 6}SCH₃
(iv) -L²-Z², dans lequel L² est une liaison directe ou un groupe espaceur, et Z² est un groupe réactif permettant à l'agent chélatant d'être lié à une molécule destinée à être marquée,
dans lequel les groupes espaceurs L¹ et L² ont une longueur de 1 à 20 liaisons entre le point de fixation et le groupe réactif, et sont formés d'un à cinq fragments, chaque fragment choisi dans le groupe constitué de phénylène, alkylène contenant 1 à 10 atomes de carbone, un éthynediyle (-C=C-), un éther (-O-), un thioéther (-S-), un disulfure (-S-S-), un amide (-C(=O)-NH-, -C(=O)-NCH₃- et -NCH₃-C(=O)-), une thio-urée (-NH-C(=S)-NH-) et un triazole,
dans lequel Z¹ ou Z² est choisi parmi azido (-N₃), alcynyle (-C≡CH), alkylène (-CH=CH₂), amino (-NH₂), amino-oxy (-O-NH₂), aldéhyde (-CHO), hydrazide (-CONHNH₂), mercapto (-SH), maléimido, dérivés activés de maléimido, isocyanato (-NCO), isothiocyanato (-NCS), diazonium (-N⁺N), bromo-acétamido, iodo-acétamido, pyridyl-2-dithio, et 4-chloro-1,3,5-triazin-2-ylamino substitué en position 6,
dans lequel deux des groupes Chrom₁, Chrom₂ et Chrom₃ ont deux ou trois substituants R¹ choisis parmi le groupe (ii) aux positions para et ortho par rapport au groupe acétylène ;
dans lequel le troisième groupe Chrom est substitué par -L²-Z².

2. Chélate selon la revendication 1 dans lequel a = b = c = 0.

3. Chélate selon la revendication 1 ou la revendication 2 dans lequel au moins l'un des groupes Chrom₁, Chrom₂ et Chrom₃ est choisi parmi la formule (IIa), (IIb) ou (IIc) : 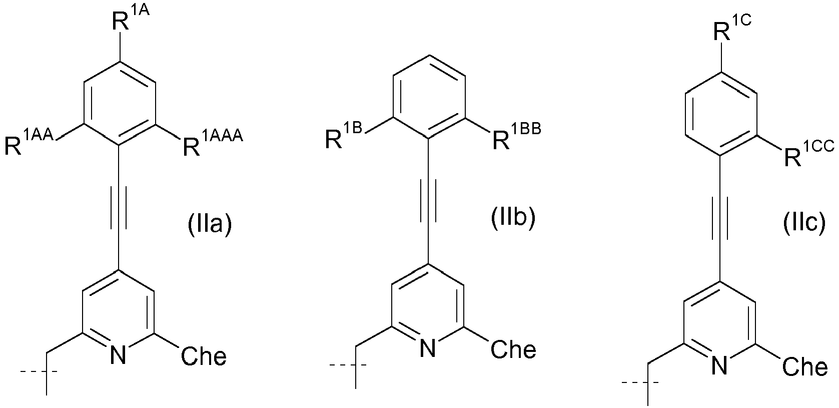 dans lequel R^{1A}, R^{1AA}, R^{1AAA}, R^{1B}, R^{1BB}, R^{1C} et R^{1CC} sont chacun indépendamment choisis parmi un groupe (ii) R¹ tel que présenté dans la revendication 1.

4. Chélate selon l'une quelconque des revendications 1 à 3 dans lequel au moins deux des groupes Chrom₁, Chrom₂ et Chrom₃ sont choisis parmi la formule (IIa), (IIb) ou (IIc) telle que définie dans la revendication 3.

5. Chélate selon l'une quelconque des revendications 1 à 4 dans lequel l'un des groupes Chrom₁, Chrom₂ et Chrom₃ est choisi parmi (IId), (IIe), (IIf) ou (IIg) : 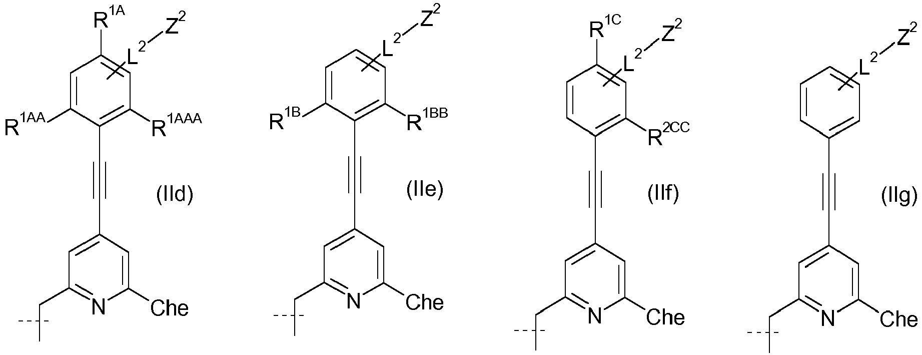 dans lequel R^{1A}, R^{1AA}, R^{1AAA}, R^{1B}, R^{1BB}, R^{1C} et R^{1CC} sont chacun indépendamment choisis parmi un groupe (ii) R¹ tel que présenté dans la revendication 1.

6. Chélate selon l'une quelconque des revendications 1 à 5 dans lequel X = -O-.

7. Chélate selon l'une quelconque des revendications 1 à 6 dans lequel les groupes L¹ ou L², lorsqu'ils sont présents, sont des liaisons directes.

8. Chélate selon l'une quelconque des revendications 1 à 7 dans lequel L¹ est un groupe espaceur choisi parmi -(CH₂)_{1 à 6}-, et *-(CH₂)_{1 à 6}Ph, dans lequel la liaison marquée * est directement reliée à l'atome d'azote de -CONR³R⁴.

9. Chélate selon l'une quelconque des revendications 1 à 8 dans lequel Z¹ ou Z² est un groupe isothiocyanato (-NCS).

10. Chélate selon l'une quelconque des revendications 1 à 9 dans lequel L² est une liaison directe et Z² est un groupe isothiocyanato (-NCS).

11. Chélate selon l'une quelconque des revendications 1 à 10 dans lequel Che est -CO₂H.

12. Chélate ayant la formule (IIIa) ou un sel de celui-ci : 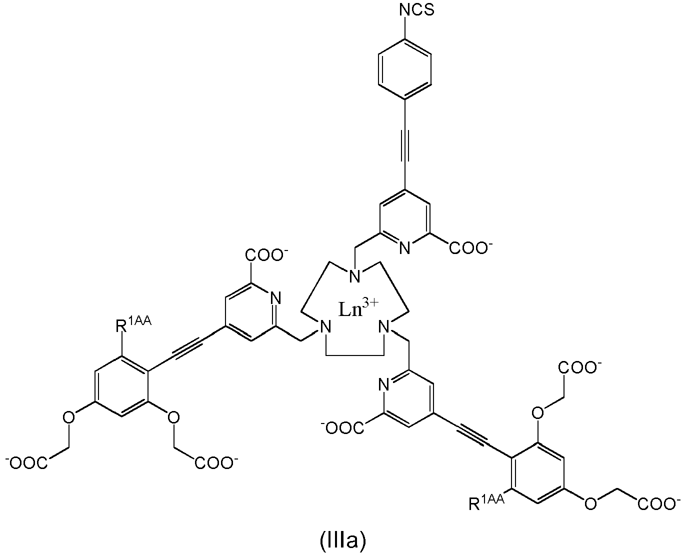 dans lequel R^{1AA} est hydrogène ou -OCH₂CO₂⁻.

13. Molécule détectable comprenant un réactif de liaison biospécifique conjugué à un chélate de lanthanide luminescent de formule (I) tel que défini dans l'une quelconque des revendications 1 à 12.

14. Molécule détectable selon la revendication 13, dans laquelle le réactif de liaison biospécifique est choisi parmi un anticorps, un antigène, un ligand de récepteur, une protéine de liaison spécifique, une sonde à ADN, une sonde à ARN, un oligopeptide, un oligonucléotide, un oligonucléotide modifié, un polynucléotide modifié, une protéine, un oligosaccharide, un polysaccharide, un phospholipide, un ANP, un stéroïde, un haptène, un médicament, un ligand de liaison à un récepteur et une lectine.

15. Molécule détectable selon la revendication 13 ou 14, dans laquelle le réactif de liaison biospécifique est un anticorps.

16. Ligand chélatant les lanthanides de formule (IV) ou un sel de celui-ci 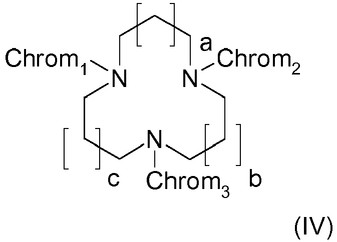 dans lequel a, b, c, Chrom₁, Chrom₂ et Chrom₃ sont tels que définis dans l'une quelconque des revendications 1 à 12.

17. Procédé de réalisation d'un essai de liaison biospécifique, ledit procédé comprenant les étapes consistant à :
a) former un biocomplexe entre un analyte et un réactif de liaison biospécifique marqué au moyen d'un chélate de lanthanide luminescent selon l'une quelconque des revendications 1 à 12 ;
b) exciter ledit biocomplexe avec un rayonnement ayant une longueur d'onde d'excitation, formant ainsi un biocomplexe excité ; et
c) détecter un rayonnement d'émission émis par ledit biocomplexe excité.

18. Utilisation d'une molécule détectable selon l'une quelconque des revendications 13 à 15 dans un essai de liaison basé sur une bioaffinité spécifique utilisant une détermination fluorométrique à résolution temporelle d'une luminescence spécifique sur la base d'une excitation d'un ou deux photons.

19. Matériau support solide conjugué avec un chélate de lanthanide luminescent selon l'une quelconque des revendications 1 à 12 ou un ligand chélatant les lanthanides selon la revendication 16.
